(19) Europäisches Patentamt
European Patent Office
Office européen des brevets

(11) **EP 4 156 198 A1**

(12) **EUROPÄISCHE PATENTANMELDUNG**

(43) Veröffentlichungstag:
**29.03.2023   Patentblatt 2023/13**

(51) Internationale Patentklassifikation (IPC):
**G16H 20/40** *(2018.01)*        **G16H 40/40** *(2018.01)*

(21) Anmeldenummer: **22195828.3**

(22) Anmeldetag: **15.09.2022**

(52) Gemeinsame Patentklassifikation (CPC):
**G16H 40/40; G16H 20/40**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **27.09.2021   DE 102021004853**
**01.10.2021   DE 102021004932**

(71) Anmelder: **Löwenstein Medical Technology S.A.**
**2557 Luxembourg (LU)**

(72) Erfinder: **Schwaibold, Matthias**
**76228 Karlsruhe (DE)**

(74) Vertreter: **Marx, Thomas**
**Löwenstein Medical Technology GmbH + Co. KG**
**IP Management**
**Kronsaalsweg 40**
**22525 Hamburg (DE)**

(54) **VORRICHTUNG UND VERFAHREN ZUR ERMITTLUNG EINER ABNUTZUNG VON BEATMUNGSGERÄTEN**

(57)     Die Erfindung betrifft eine Vorrichtung zur Bestimmung einer Nutzungsintensität eines Beatmungsgerätes, aufweisend zumindest eine Steuereinheit und eine Nutzungsuhr, die ausgebildet und eingerichtet ist, die Zeitdauer der Nutzung des Beatmungsgerätes zu erfassen. Die Vorrichtung ist dadurch gekennzeichnet, dass die Steuereinheit dazu eingerichtet und ausgebildet ist, die Zeitdauer der Nutzung des Beatmungsgerätes mit zumindest einem Abnutzungsfaktor zu verrechnen, um daraus zumindest eine Abnutzung zu ermitteln.

Figur 1

EP 4 156 198 A1

**Beschreibung**

Die Erfindung betrifft eine Vorrichtung und ein Verfahren zur Ermittlung einer Abnutzung eines Beatmungsgerätes.

Moderne Beatmungsgeräte bieten eine ganze Reihe verschiedener Therapiemöglichkeiten und Funktionen. Je nach Behandlung variiert dabei die Belastung, die auf einzelne Teile wirkt. Zur effizienten Ressourcennutzung ist es hilfreich, rechtzeitige Wartungsaktionen einzuleiten um das Gerät vor einer Beschädigung instand zu setzen.

Aus dem Stand der Technik bekannte Beatmungsgeräte bieten dabei nur sehr einfache Möglichkeiten, Intervalle zu ermitteln, in denen das Gerät gewartet wird. In der Regel wird dabei lediglich die reine Nutzungszeit aufgenommen und anhand dieser die nächste Wartung bestimmt.

Aufgabe der vorliegenden Erfindung ist daher eine Vorrichtung und ein Verfahren bereitzustellen, um eine erhöhte Betriebssicherheit von Beatmungsgeräten zu erreichen.

Zudem kann eine ausfallfreie Beatmung gewährleistet werden, wobei gleichzeitig ein schonender Materialeinsatz erreicht wird. So können Kosten und Ressourcen für Material und Transport gespart werden und die Umwelt wird zusätzlich geschont.

Die Erfindung betrifft eine Vorrichtung zur Bestimmung einer Nutzungsintensität eines Beatmungsgerätes, aufweisend zumindest eine Steuereinheit und eine Nutzungsuhr, die ausgebildet und eingerichtet ist, die Zeitdauer der Nutzung des Beatmungsgerätes zu erfassen. Die Vorrichtung ist dadurch gekennzeichnet, dass die Steuereinheit dazu eingerichtet und ausgebildet ist, die Zeitdauer der Nutzung des Beatmungsgerätes mit zumindest einem Abnutzungsfaktor zu verrechnen, um daraus zumindest eine Abnutzung zu ermitteln.

Die Zeitdauer der Nutzung des Beatmungsgerätes kann dabei direkt oder indirekt ermittelt werden. Eine direkte Ermittlung der Zeitdauer erfolgt beispielsweise durch eine Uhr oder einen Timer. Eine indirekte Ermittlung der Zeitdauer erfolgt beispielsweise unter Berücksichtigung der Abnutzung zumindest einer Komponente und/oder unter Berücksichtigung zumindest eines Abnutzungsfaktors, wobei zusätzlich die Ergebnisse einer direkten Ermittlung der Zeitdauer werden können.

In manchen Ausführungsformen kennzeichnet die Vorrichtung, dass die Steuereinheit dazu eingerichtet und ausgebildet ist, eine Mehrzahl von Abnutzungsfaktoren zu ermitteln.

In manchen Ausführungsformen kennzeichnet die Vorrichtung, dass die Steuereinheit dazu eingerichtet und ausgebildet ist, die Abnutzungsfaktoren zu einem Gesamtfaktor für das Beatmungsgerät zu verrechnen und über eine Verrechnung des Gesamtfaktors mit der Zeitdauer der Nutzung eine Gesamtabnutzung des Beatmungsgerätes zu ermitteln.

In manchen Ausführungsformen kennzeichnet die Vorrichtung, dass die Steuereinheit dazu eingerichtet und ausgebildet ist, zu einzelnen Komponenten und/oder Komponentengruppen des Beatmungsgerätes jeweils zumindest einen Abnutzungsfaktor zu ermitteln.

In manchen Ausführungsformen kennzeichnet die Vorrichtung, dass die Steuereinheit dazu eingerichtet und ausgebildet ist, die Zeitdauer der Nutzung des Beatmungsgerätes mit den jeweiligen Abnutzungsfaktoren der Komponenten und/oder Komponentengruppen zu verrechnen, um daraus eine Abnutzung der jeweiligen Komponente und/oder Komponentengruppe zu ermitteln.

In manchen Ausführungsformen kennzeichnet die Vorrichtung, dass die Steuereinheit dazu eingerichtet und ausgebildet ist, den Abnutzungsfaktor aus zumindest einem Betriebsparameter des Beatmungsgerätes zu bestimmen, wobei der zumindest eine Betriebsparameter ausgewählt wird aus Firmware-Version; Hardware-Version; Therapiemodus; Therapiedrücke; IPAP/EPAP-Rampe; Leckage; Drehzahl der Turbine/Gebläse; Drehzahländerungen der Turbine; Bremsvorgänge der Turbine; Schaltvorgänge von Ventilen; Trigger-Einstellungen; Atemfluss; Atemfrequenz; verwendete Maske; verwendetes Schlauchsystem; Anfeuchter; Umgebungstemperatur; Umgebungsluftfeuchtigkeit; Anzahl der hygienischen Aufbereitungen; Tag- oder Nachtbetrieb; Sauerstoffeinspeisung; Dauer und/oder Menge der Sauerstoffzumischung; Verwendung von Zusatzfunktionen wie Schlauchheizung und/oder Atemgasbefeuchtung; Häufigkeit von Beatmungsmanövern; Nutzung von zusätzlichen Sensoren; Status und/oder Quelle der Energieversorgung; Zeitpunkt der letzten Wartung.

In manchen Ausführungsformen kennzeichnet die Vorrichtung, dass die Steuereinheit dazu eingerichtet und ausgebildet ist, für zumindest eine Komponente ausgewählt aus Turbine; Befeuchter; Wasserkammer; Luftfilter; Schlauch; Patienteninterface; Schalldämmschäume; Elektronik; Netzteil; Akkumulator; Sensoren; Messzellen; Ventile eine Abnutzung zu ermitteln, welche unabhängig von der Gesamtabnutzung ist.

In manchen Ausführungsformen kennzeichnet die Vorrichtung, dass die Nutzungsuhr dazu eingerichtet und ausgebildet ist, neben der Zeitdauer der Nutzung des Beatmungsgerätes auch eine Standby-Zeit des Beatmungsgerätes zu erfassen.

In manchen Ausführungsformen kennzeichnet die Vorrichtung, dass die Steuereinheit dazu eingerichtet und ausgebildet ist, die Standby-Zeit des Beatmungsgerätes mit in die Gesamtabnutzung des Beatmungsgerätes zu verrechnen.

In manchen Ausführungsformen kennzeichnet die Vorrichtung, dass die Steuereinheit dazu eingerichtet und ausgebildet ist, zu jedem Abnutzungsfaktor einen durchschnittlichen Abnutzungsfaktor zu ermitteln und anhand des jeweiligen durchschnittlichen Abnutzungsfaktors eine Vorhersage bezüglich der jeweiligen Abnutzung zu treffen.

In manchen Ausführungsformen kennzeichnet die Vorrichtung, dass die Steuereinheit dazu eingerichtet und ausgebildet ist, den durchschnittlichen Abnutzungsfaktor aus dem zeitlichen Verlauf des jeweils zugrundeliegenden Abnutzungsfaktors zu ermitteln, wobei der durchschnittliche Abnutzungsfaktor eine zeitliche Gewichtung enthält.

In manchen Ausführungsformen kennzeichnet die Vorrichtung, dass die Steuereinheit dazu eingerichtet und ausgebildet ist, Abnutzungsfaktoren, welche im zeitlichen Verlauf jünger sind, für den durchschnittlichen Abnutzungsfaktor stärker zu gewichten.

In manchen Ausführungsformen kennzeichnet die Vorrichtung, dass die Steuereinheit dazu eingerichtet und ausgebildet ist, anhand der ermittelten Abnutzung und/oder der Vorhersage zur Abnutzung eine Meldung zu generieren, welche zumindest einen Hinweis auf eine Wartung enthält.

In manchen Ausführungsformen kennzeichnet die Vorrichtung, dass die Steuereinheit dazu eingerichtet und ausgebildet ist, zu erkennen, wenn die Wartung abgeschlossen ist, wobei die Meldung gelöscht wird und der Wartungszeitpunkt im Beatmungsgerät hinterlegt wird.

In manchen Ausführungsformen kennzeichnet die Vorrichtung, dass die Steuereinheit dazu eingerichtet und ausgebildet ist, anhand der ermittelten Abnutzung und/oder der Vorhersage zur Abnutzung einen optimalen Wartungszeitpunkt ermittelt und ein entsprechende Meldung generiert.

In manchen Ausführungsformen kennzeichnet die Vorrichtung, dass die Steuereinheit dazu eingerichtet und ausgebildet ist, nach einem Zeitraum, nachdem der Wartungszeitpunkt überschritten wird, eine Alarmmeldung zu generieren und bei weiterem Ausbleiben der Wartung das Beatmungsgerät und/oder bestimmte Funktionen des Beatmungsgerätes sperrt bis die Wartung durchgeführt wurde.

In manchen Ausführungsformen kennzeichnet die Vorrichtung, dass die Steuereinheit dazu eingerichtet und ausgebildet ist, nach erfolgter Wartung das gesperrte Beatmungsgerät bzw. die gesperrten Funktionen wieder zu entsperren und/oder die Alarmmeldung zu deaktivieren.

In manchen Ausführungsformen kennzeichnet die Vorrichtung, dass die Steuereinheit dazu eingerichtet und ausgebildet ist, anhand der ermittelten Abnutzung zu erkennen, ob eine Komponente des Beatmungsgerätes ausgetauscht werden muss und eine Austauschmeldung generiert, wobei die Austauschmeldung zumindest an eine entfernte Gegenstelle übermittelt wird und die Austauschmeldung an der entfernten Gegenstelle eine Aktion auslöst, welche zum Versand einer Ersatzkomponente an einen vordefinierten Ort führt.

In manchen Ausführungsformen kennzeichnet die Vorrichtung, dass die Steuereinheit dazu eingerichtet und ausgebildet ist, zusammen mit der Austauschmeldung eine Information generiert, wie die auszutauschende Komponente zu wechseln ist und automatisch erkennt, wenn die auszutauschende Komponente durch eine neue Komponente ersetzt wird.

In manchen Ausführungsformen kennzeichnet die Vorrichtung, dass die Steuereinheit dazu eingerichtet und ausgebildet ist, in periodischen Zeitabständen einen Nutzungsbericht zu erstellen, welcher zumindest eine Abnutzung umfasst und/oder eine auf der Abnutzung basierende Gebühr aufweist.

In manchen Ausführungsformen kennzeichnet die Vorrichtung, dass die Steuereinheit dazu eingerichtet und ausgebildet ist, die Gebühr aus einer Basisgebühr und einer Abnutzungsgebühr zu berechnen, wobei die Abnutzungsgebühr automatisch an die Abnutzung angepasst wird.

In manchen Ausführungsformen kennzeichnet die Vorrichtung, dass die Steuereinheit dazu eingerichtet und ausgebildet ist, einen Verbrauch an Datenvolumen als Betriebsparameter zu nutzen.

In manchen Ausführungsformen kennzeichnet die Vorrichtung, dass die Steuereinheit dazu ausgebildet und eingerichtet ist, nach dem Verbrauch eines bestimmten Datenvolumens die gesendete Datenmenge und/oder die Frequenz der Datenübertragungen verringert wird und/oder eine manuelle Sendefunktion deaktiviert wird.

Die Erfindung betrifft auch ein Verfahren zur Bestimmung einer Nutzungsintensität eines Beatmungsgerätes, wobei die Zeitdauer der Nutzung des Beatmungsgerätes erfasst wird. Das Verfahren wird dadurch gekennzeichnet, dass die Zeitdauer der Nutzung des Beatmungsgerätes mit zumindest einem Gewichtungsfaktor verrechnet wird, um daraus eine Abnutzung zu ermitteln.

In manchen Ausführungsformen kennzeichnet das Verfahren, dass eine Mehrzahl von Abnutzungsfaktoren ermittelt wird.

In manchen Ausführungsformen kennzeichnet das Verfahren, dass die Abnutzungsfaktoren zu einem Gesamtfaktor verrechnet werden und der Gesamtfaktor mit der Zeitdauer der Nutzung zu einer Gesamtabnutzung verrechnet wird.

In manchen Ausführungsformen kennzeichnet das Verfahren, dass zu einzelnen Komponenten und/oder Komponentengruppen des Beatmungsgerätes jeweils zumindest ein Abnutzungsfaktor ermittelt wird.

In manchen Ausführungsformen kennzeichnet das Verfahren, dass die Zeitdauer der Nutzung des Beatmungsgerätes mit den jeweiligen Abnutzungsfaktoren der Komponenten und/oder Komponentengruppen verrechnet werden, um daraus eine Abnutzung der jeweiligen Komponente und/oder Komponentengruppe zu ermitteln.

In manchen Ausführungsformen kennzeichnet das Verfahren, dass zu jedem Abnutzungsfaktor einen durchschnittlichen Abnutzungsfaktor ermittelt wird und anhand des jeweiligen durchschnittlichen Abnutzungsfaktors eine Vorhersage bezüglich der jeweiligen Abnutzung getroffen wird.

In manchen Ausführungsformen kennzeichnet das Verfahren, dass anhand der ermittelten Abnutzung und/oder der Vorhersage zur Abnutzung einen optimalen Wartungszeitpunkt ermittelt wird und ein entsprechende Meldung generiert wird.

In manchen Ausführungsformen kennzeichnet das Verfahren, dass nach einem Zeitraum, nachdem der Wartungszeitpunkt überschritten wird, eine Alarmmeldung genieren wird und bei weiterem Ausbleiben der Wartung das Beatmungsgerät und/oder bestimmte Funktionen des Beatmungsgerätes gesperrt werden bis die Wartung durchgeführt wurde.

In manchen Ausführungsformen kennzeichnet das Verfahren, dass nach erfolgter Wartung das gesperrte Beatmungsgerät bzw. die gesperrten Funktionen wieder entsperrt wird und/oder die Alarmmeldung deaktiviert wird.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Unter einem Beatmungsgerät ist jedwedes Gerät zu verstehen, welches einen Anwender oder Patienten bei der natürlichen Atmung unterstützt, die Beatmung des Anwenders bzw. Lebewesens (z.B. Patient und/oder Neugeborener und/oder Frühgeborene) übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiLevel-Geräte, Narkose- bzw. Anästhesiegeräte, Atemtherapiegeräte, (klinische, außerklinische oder Notfall-) Beatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen und/oder atmungsbezogenen Parametern eines Lebewesens dienen. Darunter fallen auch Geräte, welche medizinische Parameter von Patienten in Kombination mit der Atmung oder ausschließlich die Atmung betreffend, erfassen und optional verarbeiten können.

Als Patienteninterface kann, soweit nicht ausdrücklich anders beschrieben, jegliches Peripheriegerät verstanden werden, welches zur Interaktion, insbesondere zu Therapie- oder Diagnosezwecken, der Messeinrichtung mit einem Lebewesen gedacht ist. Insbesondere kann ein Patienteninterface als Maske eines Beatmungsgerätes bzw. eine mit dem Beatmungsgerät verbundene Maske verstanden werden. Diese Maske kann eine Full-Face Maske, also Nase und Mund umschließende, oder eine Nasenmaske, also eine nur die Nase umschließende Maske, sein. Auch Trachealtuben bzw. -kanülen und sogenannte Nasenbrillen können als Maske beziehungsweise Patienteninterface eingesetzt werden. In manchen Fällen kann das Patienteninterface auch ein einfaches Mundstück, beispielsweise ein Rohr, sein, durch welches das Lebewesen zumindest ausatmet und/oder einatmet.

Es ist ferner darauf hinzuweisen, dass im Zuge der Beschreibung und Ansprüche Komponenten und Komponentengruppen synonym zueinander verwendet werden. Soweit nicht explizit herausgestellt ist unter Komponente auch immer eine Komponentengruppe, also eine Zusammenfassung mehrerer Komponenten zu einer Gruppe, zu verstehen. Ebenso kann eine Komponentengruppe auch als einzelne Komponente verstanden werden.

Zur Bestimmung der Nutzungsintensität eines Beatmungsgerätes ist vorgesehen, dass die erfinderische Vorrichtung zumindest eine Steuereinheit und eine Nutzungsuhr umfasst. Über die Nutzungsuhr wird die Zeitdauer der Nutzung des Beatmungsgerätes erfasst. Die Steuereinheit ist dabei so eingerichtet, dass die Zeitdauer der Nutzung mit einem Abnutzungsfaktor verrechnet wird, um daraus eine Abnutzung zu ermitteln.

Es ist beispielsweise vorgesehen, dass die Steuereinheit mehrere Abnutzungsfaktoren ermittelt. Je nach Betriebsparameter und/oder Komponente und/oder verwendeter Funktion kann beispielsweise ein separater Abnutzungsfaktor bestimmt werden. Insbesondere für verschiedene Komponenten des Beatmungsgerätes und/oder Komponentengruppen sind separater Abnutzungsfaktoren vorgesehen.

Die Steuereinheit ist beispielsweise auch dazu eingerichtet und ausgebildet ist die Zahl und/oder Dauer der Ladezyklen oder die Kapazität oder den Innenwiderstand des Akkumulators oder andere Indikatoren für die Nutzung des Akkumulators zu ermitteln und für die Ermittlung zumindest eines Abnutzungsfaktors zu berücksichtigen.

Über die Steuereinheit werden die separaten Abnutzungsfaktoren zu einem Gesamtfaktor verrechnet. Es kann dabei vorgesehen sein, dass unterschiedliche Abnutzungsfaktoren mit unterschiedlichen Gewichtungen in den Gesamtfaktor verrechnet werden. Der Gesamtfaktor wird von der Steuereinheit dazu verwendet, um über eine Verrechnung mit der Zeitdauer der Nutzung eine Gesamtabnutzung für das Beatmungsgerät zu bestimmen. Die Gesamtabnutzung kann dabei repräsentativ für einen allgemeinen Zustand des Beatmungsgerätes sein. Die Gesamtabnutzung kann so vorgesehen sein, dass sie in einer Zahl und/oder Information einen allgemeinen Status der Abnutzung des Beatmungsgerätes wiedergibt. Einem Anwender und/oder Betreuer wird so beispielsweise ermöglicht, anhand einer einfachen Zahl und/oder Information den Zustand des Beatmungsgerätes zu beurteilen.

Eine einfache Verrechnung von Gesamtfaktor und Zeitdauer der Nutzung kann beispielsweise über eine Multiplikation erfolgen, sodass die Abnutzung in Form einer Zeitdauer angegeben wird. In manchen Ausführungsformen kann vorgesehen sein, weitere Faktoren einzuführen, beispielsweise um eine einheitslose Kennzahl für die Abnutzung zu erzeugen.

Der Gesamtfaktor kann alternativ oder ergänzend auch einen Durchschnitt, gegebenenfalls gewichtet, aller einzelnen Abnutzungsfaktoren und/oder Abnutzungen wiedergeben. Es kann dazu vorgesehen sein, dass die Abnutzungsfaktoren normiert werden, sodass sie miteinander vergleichbar werden. Beispielsweise kann vorgesehen sein, dass die Abnutzung als ein Anteil und/oder Prozentsatz wiedergegeben wird, wobei 100% einer vollständigen Abnutzung entsprechen kann und/oder das Erreichen eines Wartungslimits, also einem Zeitpunkt bei dem das Beatmungsgerät und/oder die betroffene Komponente gewartet werden sollte.

Alternativ oder ergänzend kann vorgesehen sein, dass die Gesamtabnutzung die Abnutzung der Komponente wiedergibt, deren Abnutzung am höchsten ist.

Auch kann, zusammen mit der Gesamtabnutzung, immer die Abnutzung der Komponenten mit der höchsten und mit der niedrigsten Abnutzung angezeigt bzw. ausgegeben werden.

Für einzelne Komponenten und/oder Komponentengruppen kann vorgesehen sein, separate Abnutzungen zu bestimmen. Dazu wird den Komponenten jeweils zumindest ein Abnutzungsfaktor zugeordnet, welcher mit der Zeitdauer der Nutzung verrechnet wird um die Abnutzung zu bestimmen. Die Abnutzung der einzelnen Komponenten kann dabei unabhängig von der Gesamtabnutzung bestimmt werden.

Alternativ oder ergänzend kann vorgesehen sein, dass die Abnutzung der einzelnen Komponenten mit in die Gesamtabnutzung verrechnet wird. Beispielsweise kann die Gesamtabnutzung anteilig durch eine Verrechnung des Gesamtfaktors mit der Zeitdauer der Nutzung und anteilig durch eine Verrechnung der Abnutzung einzelner Komponenten berechnet werden.

Es ist beispielsweise vorgesehen, dass in die Abnutzungsfaktoren verschiedene Betriebsparameter des Beatmungsgerätes einfließen können, beispielsweise als Teilfaktoren. Je nach Abnutzungsfaktor kann dabei eine andere Berechnungsgrundlage vorliegen.

Beispielsweise kann für manche Abnutzungsfaktoren vorgesehen sein, dass je nach Wert des Betriebsparameters eine zeitliche Akkumulierung eines dem Wert zugeordneten Teilfaktors genutzt wird. Wird beispielsweise einem eingestellten und/oder gemessenen Betriebsparameter ein Wert a der Teilfaktor 2 zugeordnet und besteht dieser Wert a für 10 Zeiteinheiten, so ergibt der entsprechende Abnutzungsfaktor den Wert 20. Es kann für alle Abnutzungsfaktoren vorgesehen sein, dass in der Berechnung jeweils ein Einheitsfaktor vorgesehen ist, welcher den Abnutzungsfaktor einheitenlos macht. Fließen mehrere Teilfaktoren in einen Abnutzungsfaktor ein, so kann beispielsweise vorgesehen sein, dass die Teilfaktoren aufaddiert und/oder miteinander multipliziert werden. Auch eine Gewichtung der Teilfaktoren kann vorgesehen sein. In manchen Ausführungsformen kann zudem vorgesehen sein, dass Betriebsparameter in verschiedene Abnutzungsfaktoren mit unterschiedlichen Teilfaktoren eingerechnet werden. So kann berücksichtigt werden, wenn verschiedene Komponenten, denen unterschiedliche Abnutzungsfaktoren zugeordnet sind, in unterschiedlicher Weise von denselben Betriebsparametern bezüglich der Abnutzung betroffen sind.

Eine Berechnung der Gesamtabnutzung kann beispielsweise gemäß

$$GA = TG \cdot GF$$

erfolgen, wobei GA die Gesamtabnutzung, TG die Zeitdauer der Nutzung des Beatmungsgerätes und GF der Gesamtfaktor ist. Der Gesamtfaktor GF berechnet sich beispielsweise über

$$GF = \sum(AF_i \cdot G_i)$$

wobei $AF_i$ der Abnutzungsfaktor und G der zugehörige Gewichtungsfaktor ist. Die einzelnen Abnutzungsfaktoren $AF_i$ folgen beispielsweise aus

$$AF_i = \sum BP_i$$

und/oder

$$AF_i = BP_1 \cdot BP_2 \cdot ... \cdot BP_i$$

wobei BP jeweils die Teilfaktoren bezeichnet, welche bestimmten Betriebsparametern zugeordnet sind. In die Berechnung der Abnutzungsfaktoren werden dabei jeweils nur die Teilfaktoren bzw. Betriebsparameter mit einbezogen, welcher den jeweiligen Abnutzungsfaktoren zugeordnet sind.

In manchen Ausführungsformen kann eine Berechnung der Gesamtabnutzung GA auch über

$$GA = \sum(K_i \cdot G_i)$$

erfolgen, wobei K die Abnutzung einzelner Komponenten ist. Die Abnutzung einzelner Komponenten K kann beispielsweise anhand einer Summe der mit der jeweiligen Komponente verbundenen Abnutzungsfaktoren multipliziert mit der Zeitdauer der Nutzung der jeweiligen Komponente erfolgen.

Ein Betriebsparameter kann zum Beispiel der gewählte Therapiemodus sein. Dabei wird beispielsweise zwischen einem CPAP und einem BiLevel-Betrieb unterschieden. Auch weitere Therapiemodi können berücksichtig werden. Beispielhaft sind hier eine invasive und nicht-invasive Beatmung, die (bspw. antizyklische) Servoventilation, eine Mundstückbeatmung, nasale Highflow-Therapie, autoCPAP-Modus, allgemein automatische Beatmungsmodi wie mit auto-EPAP (automatischer exspiratorischer, positiver Atemwegsdruck) oder Zielvolumensteuerung zu nennen. Beispielsweise ist für jeden Therapiemodus ein eigener Teilfaktor vorgesehen. Der Teilfaktor für jeden Therapiemodus kann dabei beispielsweise gewisse Einstellungen pauschal berücksichtigen. Ist für einen Therapiemodus eine häufige Änderung des Beatmungsdruckes vorgesehen, kann für den Therapiemodus ein höherer Teilfaktor vorgesehen sein, als für einen Therapiemodus mit weniger Änderungen des Beatmungsdruckes. Eine Erhöhung des Teilfaktors, welcher einem Therapiemodus zugeordnet ist, kann auch berücksichtigen, ob zusätzliche Funktionen wie Befeuchtung und/oder Erwärmung des Atemgases vorgesehen ist. In manchen Ausführungsformen ist dabei vorgesehen, dass einem Therapiemodus ein voreingestellter Teilfaktor zugeordnet ist.

Ein anderer Betriebsparameter kann beispielsweise die Therapiedrücke berücksichtigen. Beispielsweise stellen höhere Drücke eine größere Belastung für das Beatmungsgerät und/oder einzelne Komponenten dar. Entsprechend kann vorgesehen sein, dass ein höherer Therapiedruck auch zu einem höheren Teilfaktor bzw. Abnutzungsfaktor führt. In manchen Ausführungsformen kann beispielsweise auch berücksichtigt werden, dass verschiedene Komponenten unterschiedliche optimale Belastungsbereiche bezüglich des Therapiedrucks aufweisen. Entsprechend kann vorgesehen sein, je nach Komponente den Einfluss des Therapiedrucks auf den Abnutzungsfaktor entsprechend anders zu berücksichtigen. Der Therapiedruck kann beispielsweise über einen Drucksensor des Beatmungsgerätes gemessen und aufgezeichnet werden.

Ein weiterer möglicher Betriebsparameter können Einstellungen bezüglich einer Rampe zwischen dem inspiratorischen Druckniveau (IPAP) und dem exspiratorischen Druckniveau (EPAP) sein. Beispielsweise kann hier die Steilheit der Rampe miteinbezogen werden. Eine größere Steilheit kann zum Bespiel mit einem größeren Faktor eingehen als eine vergleichsweise flache Rampe. Es kann zum Beispiel vorgesehen sein, dass die Steilheit als Teilfaktor für den Abnutzungsfaktor der Turbine/des Gebläses bzw. des entsprechenden Motors einbezogen wird. Eine größere Steilheit der Rampe bedeutet für den Motor dabei, dass die Drehzahl stärker beschleunigt bzw. abgebremst werden muss, was zu einer höheren Belastung und damit auch einer schnelleren Abnutzung des Motors führt. Der Betriebsparameter der Rampe kann dabei beispielsweise als pauschaler Faktor verwendet werden, welcher für den gesamten Therapiezeitraum, für welchen die Rampe eingestellt ist, konstant bleibt. Ist das Beatmungsgeräte dazu eingerichtet die Rampe entsprechend der Atmung des Patienten dynamisch anzupassen, so kann der Betriebsparameter Rampe auch als dynamischer Faktor eingerechnet werden. Dazu wird bei jedem Wechsel zwischen EPAP und IPAP ein entsprechender Faktor für die Rampe bestimmt und anteilig eingerechnet.

Auch kann eine gemessene Leckage als Betriebsparameter in Abnutzungsfaktoren einfließen. Eine höhere Leckage kann dabei beispielsweise als höherer Faktor gelten, da durch eine höhere Leckage ein höherer Gasfluss erzeugt werden muss, um die Leckage auszugleichen. Der Betriebsparameter der Leckage kann optional auch mit in die Betrachtung eines Atemgasbefeuchters einbezogen werden, wobei eine höhere Leckage gleichzeitig zu einer höheren Abnutzung des Atemgasbefeuchters beiträgt, da pro Zeiteinheit eine größere Menge an Atemgas befeuchtet werden muss als bei einer geringeren Leckage.

Ein weiterer Betriebsparameter kann die Drehzahl der Turbine bzw. des Gebläses darstellen. Beispielsweise wird durch die Steuereinheit eine Drehzahl vorgegeben und/oder gemessen. Die Drehzahl der Turbine kann insbesondere in den Abnutzungsfaktor, welcher der Turbine und/oder dem Motor der Turbine zugeordnet ist, einbezogen werden. In manchen Ausführungsformen ist dabei vorgesehen, dass Drehzahlbereiche festgelegt werden, wobei den Drehzahlbereichen jeweils entsprechende Teilfaktoren zugeordnet werden.

Zusammen oder unabhängig mit der Drehzahl kann auch vorgesehen sein, dass Drehzahländerungen als Betriebsparameter mit in die Abnutzungsfaktoren einbezogen werden. Eine häufige und/oder größere Änderung der Drehzahl, beispielsweise durch Beschleunigung oder Abbremsen, kann beispielsweise als größere Belastung gewertet werden und wird mit einem größeren Faktor in die Abnutzung bzw. die Abnutzungsfaktoren einbezogen.

Auch die Anzahl an Schaltvorgängen von Ventilen kann als Betriebsparameter genutzt werden, um Abnutzungsfaktoren zu bestimmen. Beispielsweise umfasst die Vorrichtung eine Zähleinheit, welche die Anzahl der Schaltvorgänge je Ventil erfasst. Alternativ oder ergänzend kann vorgesehen sein, dass die Zeit zwischen zwei Schaltvorgängen desselben Ventils miteinbezogen werden. Unterschreitet die Zeit zwischen zwei Schaltvorgängen beispielsweise einen bestimmten Schwellenwert, kann hierfür ein gegenüber zwei weiter auseinanderliegenden Schaltvorgängen erhöhter Einfluss einberechnet werden. Der Einfluss kann beispielsweise auch akkumulierend berechnet werden. Werden beispielsweise mehrere Schaltvorgänge registriert, zwischen denen die Zeit einen Schwellenwert unterschreitet so erhöht sich der Einfluss bzw. der Teilfaktor immer weiter. Dadurch wird berücksichtigt, dass schnelle Schaltungen hintereinander eine größere Belastung für das Ventil darstellen.

Ergänzend oder alternativ können auch Trigger-Einstellungen als Betriebsparameter angesehen werden. Dabei kann beispielsweise die Sensitivität eine Rolle spielen. Eine höhere Sensitivität kann beispielsweise zu einer höheren Rate von falschen Triggern führen, also Situationen in denen das Beatmungsgerät zwischen Inspiration und Exspiration umschaltet obwohl eine solche Umschaltung nicht nötig ist. Daraus resultiert beispielsweise, dass der Patient für einen Zeitraum gegen das Beatmungsgerät atmet und somit eine höhere Belastung auf das Gerät ausübt. Da gleiches auch für eine zu geringe Sensitivität des Triggers gelten kann, kann vorgesehen sein, auch die Anzahl von Fehltriggern, also Umschaltungen zum falschen Zeitpunkt und/oder ausgebliebene Umschaltungen, mit in die Abnutzungsfaktoren einzubeziehen.

Auch ein gemessener Atemgasfluss kann als Betriebsparameter gelten. Dabei kann beispielsweise ein hoher Atemgasfluss zu einem höheren Abnutzungsfaktor führen. Ebenso ist auch die Atemfrequenz des Patienten als Betriebsparameter berücksichtigt werden. Es kann vorgesehen sein, dass der Atemgasfluss über einen Zeitraum gemittelt wird, beispielsweise über eine Stunde und/oder einen Tag. Aus dem Mittelwert kann sich unter anderem der Fortschritt der Abnutzung ermitteln und/oder zumindest abschätzen lassen. Auch die Änderung des Atemgasflusses kann als Betriebsparameter genutzt werden, beispielsweise jede Änderung einzeln und/oder als Mittelwert bzw. Median über ein Zeitintervall. Über die Änderung des Atemgasflusses lassen sich so beispielsweise Beschleunigungs- und/oder Abbremsvorgänge der Turbine bzw. des Gebläses miteinbeziehen.

Als weiterer Betriebsparameter kann das verwendete Zubehör und Peripherie in die Abnutzungsfaktoren miteinberechnet werden. In manchen Ausführungsformen ist das Beatmungsgerät beispielsweise auf den Betrieb mit bestimmten Zubehör wie Masken, Schläuchen und/oder Befeuchtern optimiert. Davon abweichendes Zubehör kann beispielsweise eine schnellere Abnutzung einzelner Komponenten bewirken und gegebenenfalls auch selbst einer höheren Abnutzung unterliegen. Entsprechend können mit dem Zubehör individuelle Teilfaktoren vergeben werden. In manchen Ausführungsformen ist das Beatmungsgerät dazu eingerichtet angeschlossenes Zubehör automatisch zu erkennen und/oder zumindest zu erkennen ob ein optimiertes Zubehör angeschlossen ist oder nicht. Auch kann das Beatmungsgerät dazu eingerichtet sein, bestimmte Eigenschaften des angeschlossenen Zubehörs zu erkennen und/oder zu messen. Beispielsweise kann das Beatmungsgerät den Widerstand des angeschlossenen Schlauchsystems und/oder des angeschlossenen Patienteninterface zu bestimmen. Ein höherer gemessener Widerstand kann dabei mit einem höheren Faktor einfließen.

Unter anderem im Zusammenhang mit angeschlossenem Zubehör kann auch ein pneumatischer Widerstand als Betriebsparameter in die Abnutzung verschiedener Komponenten einfließen. Ein höherer pneumatischer Widerstand stellt beispielsweise eine höhere Belastung für die Turbine bzw. das Gebläse dar und kann somit zu einer Erhöhung der jeweiligen Abnutzungsfaktoren führen.

Ergänzend oder alternativ kann vorgesehen sein, dass der Betrieb angeschlossener Atemgasbefeuchtung und/oder Atemgaserwärmung als Betriebsparameter mit in die Abnutzungsfaktoren einbezogen wird. Hier kann beispielsweise die gemessene und/oder eingestellte Feuchtigkeit bzw. Temperatur eine Rolle spielen. Beispielsweise kann es einen optimalen Temperatur- und/oder Feuchtigkeitsbereich geben, wobei ein Betrieb außerhalb dieser Bereiche eine höhere Abnutzung zu erwarten ist und entsprechend die Abnutzungsfaktoren und/oder Teilfaktoren höher gewertet werden.

Neben der Atemgasbefeuchtung und Atemgastemperatur können auch Umgebungsbedingungen wie Temperatur, Feuchte und/oder Luftdruck als Betriebsparameter berücksichtig werden. Auch hier können beispielsweise ideal Bereiche definiert sein, in welchen die Teilfaktoren bzw. Abnutzungsfaktoren entsprechend niedriger gewertet werden als bei einem Betrieb außerhalb dieser Bereiche.

Auch die Luftqualität, etwa der Gehalt an Staub und/oder anderen Partikeln, kann als Betriebsparameter genutzt werden. Ein hoher Staubgehalt der Umgebungsluft kann beispielsweise dazu beitragen, dass die Filter des Beatmungsgerätes schneller belegt bzw. verstopft sind. Auch kann sich ein erhöhter Staubgehalt auf die Lager der Turbine auswirken, etwa durch eine Akkumulation von Staubpartikeln auf dem Lager und/oder in Schmierstoffen. Auch kann der Staub sich auf und/oder in verschiedenen anderen Komponenten ablagern, etwa in Schaumstoffen, welche der Schalldämmung dienen.

Auch die Anzahl der Patientenwechsel bzw. Vorgänge zur hygienischen Aufbereitung können relevante Betriebsparameter sein. Verschiedene hygienische Aufbereitungsarten wirken sich dabei unterschiedlich auf die Abnutzung aus. Es kann beispielsweise vorgesehen sein, dass die Steuereinheit automatisch und/oder semiautomatisch erkennt, welche Aufbereitungsmethode genutzt wird und gegebenenfalls auch die Dauer der Aufbereitung erkennt. Alternativ oder ergänzend kann vorgesehen sein, dass ein Benutzer Informationen und/oder Daten zu der hygienischen Aufbereitung, beispielswiese die Art und Dauer der Aufbereitung, im Beatmungsgerät hinterlegt. In manchen Ausführungsformen verfügt das Beatmungsgerät eine Auswahl an Aufbereitungsmethoden, welche vom Benutzer ausgewählt werden können.

Auch die Verwendung verschiedener voreingestellter Programme, beispielsweise einem Tag- und Nachbetrieb zwischen dem der Anwender/Patient wählen kann, kann als Betriebsparameter in die Abnutzungsfaktoren einfließen. Denkbar ist dabei eine Verrechnung des Verhältnisses zwischen den Programmen. Es kann auch vorgesehen sein, dass die Nutzungsdauer bzw. die Abnutzung anhand der Programme einzeln ermittelt wird und dann für jede Komponente zu einer Gesamtabnutzung aufaddiert wird. Beispielsweise ermittelt sich die Abnutzung einer Komponente dann daraus, dass die Abnutzung aus den einzelnen Programmen, etwa dem Tag- und Nachtbetrieb, zusammengerechnet werden.

Es kann auch vorgesehen sein, dass eine Abnutzung eines Displays unter anderem anhand des Tag- und Nachtbetriebs bestimmt wird. Hierbei ist beispielsweise entscheidend, mit welcher Helligkeit das Display betrieben wird. Typischerweise wird die Displayhelligkeit und/oder die allgemeine Nutzung des Displays, für einen Nachtbetrieb stark heruntergeregelt, sodass eine geringere Belastung des Displays resultiert, im Nachtbetrieb also eine geringere Abnutzung zu verzeichnen ist.

Es kann alternativ oder ergänzend auch vorgesehen sein, dass die Sauerstoffeinspeisung und/oder -zumischung als Betriebsparameter mit in die Abnutzungsfaktoren eingerechnet wird. Beispielsweise geht dabei mit ein, ob eine Sauerstoffzumischung aktiviert ist oder nicht und welche Menge und über welchen Zeitraum Sauerstoff zugemischt wird. Beispielsweise geht eine höhere Menge und/oder höhere Dauer mit einem höheren Teilfaktor in die Abnutzungsfaktoren ein.

Weitere Betriebsparameter sind in der Verwendung von Zusatzfunktionen wie Schlauchheizung, Befeuchtung und/oder Komfortfunktionen (Autostart, Startrampe, Stopprampe, etc.) zu finden. Wird beispielsweise eine Start-/Stopprampe genutzt, kann dies mit einer Verringerung des Teilfaktors und/oder des Abnutzungsfaktors bedacht werden, da das Beatmungsgerät langsam in den endgültigen Betriebszustand übergeht statt eines "plötzlichen" Starts der Beatmung. Eine Autostartfunktion hingegen kann als erhöhend auf den Teilfaktor/Abnutzungsfaktor wirken, da hier gegebenenfalls eine Grundaktivität des Beatmungsgerätes vorausgesetzt wird und/oder verschiedene Sensoren aktiviert sein müssen. Die Aktivierung einer Schlauchheizung wirkt sich beispielsweise besonders auf den Abnutzungsfaktor des Schlauchsystems aus, beispielsweise kann ein idealer Temperaturbereich für das Schlauchsystem vorgesehen sein und ein Betrieb außerhalb dieses Temperaturbereichs führt zu einer schnelleren bzw. höheren Abnutzung, was sich im Abnutzungsfaktor wiederspiegelt.

Auch die Häufigkeit bestimmter Manöver wie einer Hustenhilfe und oder einem Hustenstoß durch den Patienten kann als Betriebsparameter vorgesehen sein. Solche Spezialmanöver üben dabei eine zusätzliche Belastung auf das Beatmungsgerät aus, sodass diese beispielsweise mit einer Erhöhung des Abnutzungsfaktors einhergehen können.

Die Nutzung von Filtern, wie beispielsweise Bakterienfiltern, sind in manchen Ausführungsformen ebenfalls als Betriebsparameter vorgesehen, welche mit in die Abnutzungsfaktoren einbezogen werden. Beispielsweise kann vorgesehen sein, Filter mit einem den Abnutzungsfaktor verringernden Einfluss einzuberechnen, wobei der entsprechende Teilfaktor von der Abnutzung des Filters selbst abhängen kann. Beispielsweise kann die Steuereinheit dazu eingerichtet sein, die Beladung des Filters abzuschätzen und/oder zu messen und bei einer höheren Beladung, beispielsweise bei Überschreiten eines Schwellenwertes, den Teilfaktor für den Filter zu erhöhen. Die Beladung des Filters kann beispielsweise über den Fluss und den damit zusammenhängend zu erzeugenden Druck und/oder der nötigen Drehzahl des Turbinenmotors und/oder einen pneumatischen Widerstand bestimmt werden. In manchen Ausführungsformen bewirkt eine höhere Beladung des Filters einen geringeren Fluss, welcher dadurch kompensiert wird, dass ein höherer Druck ausgeübt werden muss um den vorgesehenen Fluss zu erreichen.

Die Nutzungszeit von zusätzlichen Sensoren, wie z.B. $CO_2$, $SpO_2$, $O_2$, $FiO_2$, Feuchtigkeit, Temperatur kann ebenfalls als Betriebsparameter angesehen werden. Beispielweise kann vorgesehen sein, dass die Nutzungszeit direkt als Abnutzungsfaktor genutzt wird und/oder auch für die Sensoren direkt eine Abnutzung darstellt. Die Abnutzung eines Sensors kann beispielsweise nur von der Nutzungszeit dieses Sensors abhängen. So kann die Abnutzung des Sensors auch direkt, gegebenenfalls gewichtet, in die Gesamtabnutzung eingehen. Alternativ oder ergänzend kann auch die Zahl und/oder Dauer der Nutzungen des $FiO_2$-Sensors in den oder die Abnutzungsfaktoren einbezogen werden.

Ein weiterer Betriebsparameter, welcher bei der Berechnung bzw. Ermittlung der Abnutzungsfaktoren miteinbezogen werden kann bezieht sich auf die Energieversorgung. Beispielsweise kann hier der Energieverbrauch berücksichtigt werden. Führt beispielsweise ein höherer Energieverbrauch zu einer höheren Belastung, beispielsweise des Netzteils und/oder eines Akkumulators, so kann der Abnutzungsfaktor entsprechend höher ausfallen. Die Steuereinheit ist beispielsweise dazu eingerichtet und ausgebildet ist die Zahl und/oder Dauer der Ladezyklen oder die Kapazität oder den Innenwiderstand des Akkumulators oder andere Indikatoren für die Nutzung des Akkumulators zu ermitteln und für die Ermittlung zumindest eines Abnutzungsfaktors zu berücksichtigen. Indikatoren für die Nutzung des Akkumulators sind insbesondere die im Folgenden genannten: Alternativ oder ergänzend kann auch die Zahl und/oder Dauer der Ladezyklen des Akkumulators in den oder die Abnutzungsfaktoren einbezogen werden. Es kann auch vorgesehen sein, dass der Zeitpunkt des Ladebeginns, etwa mit Hinblick auf den Ladezustand des Akkumulators, miteinbezogen wird. Beispielsweise kann die Zahl der Ladezyklen auch über den Ladezustand des Akkumulators gewichtet werden. Ein höherer Ladezustand bei Beginn der Ladung kann etwa einen höheren Abnutzungsfaktor, insbesondere für den Akkumulator, bedeuten als ein eher niedriger Ladezustand. Alternativ oder ergänzend kann auch die Kapazität des Akkumulators in die Abnutzungsfaktoren einbezogen werden. Die Kapazitätsmessung erfolgt dabei unter Berücksichtigung der maximal zulässigen Lade-/Entladeströme des Akkumulators. Insbesondere kann auch die Kapazität beim Laden für die Ermittlung des Abnutzungsfaktors ermittelt werden.

Alternativ oder ergänzend kann auch der Innenwiderstand des Akkumulators in die Abnutzungsfaktoren einbezogen werden. Die Messung des Innenwiderstands erfolgt dabei unter Berücksichtigung der maximal zulässigen Lade-/Entladeströme des Akkumulators. Insbesondere kann auch der Innenwiderstand beim Laden für die Ermittlung des Abnutzungsfaktors ermittelt werden. Alternativ oder ergänzend kann auch das kalendarische Alter des Akkumulators in die Abnutzungsfaktoren einbezogen werden. Alternativ oder ergänzend kann auch die Anzahl der Ladezyklen gezählt werden und in den Abnutzungsfaktor einbezogen werden. Alternativ oder ergänzend kann auch der Ladezustand zu Beginn und zum Ende des Ladezyklus bestimmt werden, um zu ermitteln wie tief der Akkumulator entladen wie stark er wieder aufgeladen wurde und das Ergebnis in den Abnutzungsfaktor einbezogen werden. Alternativ oder ergänzend kann auch die Stärke der Ladeströme / Entladeströme und/oder die zur Verfügung gestellte Leistung Ergebnis in den Abnutzungsfaktor einbezogen werden.

Auch die Umgebungsbedingungen bei Nutzungsvorgängen und Ladungsvorgängen, wie die Temperatur oder Luftfeuchte können ermittelt werden und in den Abnutzungsfaktor einbezogen werden.

Alternativ oder ergänzend kann auch Verhalten beim aktuellen Ladezyklus ermittelt werden und in den Abnutzungsfaktor einbezogen werden, beispielsweise als Innenwiderstand des Akkumulators und/oder Erwärmungsverhalten abhängig vom Ladestrom und/oder verbleibende Akkumulatorkapazität und/oder Strom-/Spannungskennlinie während der Ladung.

Alternativ oder ergänzend kann auch eine relative Veränderung von Eigenschaften des Akkumulators zum Neuzustand ermittelt werden und in den Abnutzungsfaktor einbezogen werden, wobei dazu die Eigenschaften des Akkumulators im Neuzustand bestimmt und gespeichert werden.

Abnutzungsfaktoren können beispielsweise auch die Hardware-Version einzelner Komponenten beinhalten. Beispielsweise kann eine neuere Hardware-Version im Betrieb optimiert sein und/oder wartungsärmer sein, wodurch eine geringere Abnutzung zu beobachten sein kann. Ergänzend oder alternativ kann auch bedacht werden, dass verschiedene Hardware-Versionen auch verschiedene Betriebsbereiche aufweisen, also Bereiche in denen eine geringere Abnutzung als außerhalb dieser Bereiche zu beobachten ist.

In manchen Ausführungsformen kann beispielsweise auch das Material der Komponenten berücksichtigt werden. Beispielsweise kann zwischen langlebigen, normalen und/oder kurzlebigen Komponenten unterschieden werden. Entsprechend kann beispielsweise vorgesehen sein, dass die Materialien der Komponenten mit in die Abnutzungsfaktoren einbezogen werden. In manchen Ausführungsformen kann auch vorgesehen sein, dass die maximale Abnutzung und/oder Zeitdauer der Nutzung bis zur Wartung und/oder Austausch der Komponente entsprechend angepasst wird. Kurzlebige Komponenten müssen sich beispielsweise nach einer kürzeren Nutzungszeit einer Wartung und/oder Austausch unterziehen. Das Material der Komponenten spielt beispielsweise bei den verwendeten Masken, Schläuchen, Sensoren, Akkumulators und/oder Gebläsen/Atemgasquellen eine Rolle.

Auch die Firmware-Version des Beatmungsgerätes bzw. einzelner Komponenten kann sich in den Abnutzungsfaktoren niederschlagen. Neuere Firmware kann beispielsweise hinsichtlich der Steuerung optimiert sein, sodass eine geringere Belastung von einzelnen Komponenten bzw. dem Beatmungsgerät realisiert werden kann und entsprechend ein niedrigerer Abnutzungsfaktor und/oder Teilfaktor angesetzt werden kann.

Es kann auch vorgesehen sein, dass der Verbrauch von Datenvolumen, z.B. für den Versand von Daten per Mobilfunk und/oder Bluetooth und/oder WLAN, als Betriebsparameter angesehen wird und/oder in Abnutzungsfaktoren mit einfließt. Beispielsweise ist ein bestimmtes Datenvolumen pro Zeiteinheit, etwa pro Monat, vorgesehen. Ist dieses vorgesehene Datenvolumen aufgebraucht können beispielsweise bestimmte Übertragungskanäle und/oder die Sendefunktion allgemein gesperrt werden. Auch kann über bereits verbrauchtes Datenvolumen eine Vorhersage erstellt werden, ob das vorgesehene Datenvolumen ausreichend ist. Wird vorhergesagt, dass das vorgesehene Datenvolumen nicht ausreichend ist, wird beispielsweise eine Meldung ausgegeben und/oder die gesendete Datenmenge reduziert und/oder die Frequenz der Datenübertragung verringert. Es kann zusätzlich vorgesehen sein, dass eine manuelle Sendefunktion deaktiviert wird. Auch kann vorgesehen sein, dass ein Sicherheitsdatenvolumen vorgehalten wird, sodass eine Übermittlung von Alarmen und/oder Notfall-Meldungen dennoch möglich bleibt.

Ist eine maximale Abnutzung des Datenvolumens erreicht, also das verfügbare Datenvolumen aufgebraucht, so kann hier eine automatische Art der Wartung bzw. Austausch erfolgen. Eine Wartung bzw. Austausch sieht dabei beispielsweise vor, dass im Falle von Prepaid-Datenvolumen, also ein Datenvolumen welches im Voraus gebucht wird, das Datenvolumen aufgeladen wird. Beispielsweise wird dazu eine zusätzliche Gebühr fällig, welche auf eine Gesamtgebühr aufgerechnet wird. Ist ein Tarif-Datenvolumen vorgesehen, kann vorgesehen sein, dass die Steuereinheit eine automatische Änderung des Tarifs durchführt, beispielsweise auf einen Tarif mit höheren verfügbaren Datenvolumen. In manchen Ausführungsformen kann dabei vorgesehen sein, dass die Änderung durch einen Nutzer und/oder Betreuer bestätigt werden muss. In manchen Ausführungsformen kann auch vorgesehen sein, dass ein Kontingent an Datenvolumen für eine Mehrzahl an Beatmungsgeräten bereitgestellt wird. Bei einem Verbrauch des vorgesehenen Datenvolumens eines Beatmungsgerätes kann dann das verbleibende Datenvolumen aus dem Gesamtkontingent umverteilt werden beziehungsweise zusätzliches Datenvolumen freigegeben werden, etwa durch Abziehen von Datenvolumen von Geräten mit geringerem Verbrauch an Datenvolumen.

Ein weiterer Betriebsparameter kann auch die Anzahl oder Häufig der Wartung des Beatmungsgeräts und/oder einzelner Komponenten sein. Zum Beispiel kann ein häufiges Öffnen des Beatmungsgerätes zu einer höheren Belastung des Gehäuses führen. Auch das Ein- und Ausbauen einzelner Komponenten kann sich auf die Abnutzung des Beatmungsgerätes auswirken.

Die Abnutzung kann beispielsweise für das Beatmungsgerät komplett ermittelt werden und/oder separat für einzelne Komponenten wie Turbine, Befeuchter, Wasserkammer, Luftfilter, Schlauch, Patienteninterface, Schäume, Elektronik, Netzteil, Sensoren, Messzellen etc.

Hierbei kann auch vorgesehen sein, dass jeder Komponente ein oder mehrere Abnutzungsfaktoren zugeordnet werden. Die Abnutzungsfaktoren bestimmen dabei beispielsweise wie stark sich verschiedene Betriebsparameter auf die jeweilige Komponente auswirken. Dabei kann auch angedacht sein, dass die gleichen Betriebsparameter in unterschiedliche Abnutzungsfaktoren unterschiedlich einberechnet, beispielsweise gewichtet, werden. Alternativ oder ergänzend können die Betriebsparameter mit unterschiedlichen Teilfaktoren in die Abnutzungsfaktoren einbezogen werden.

Ergänzend kann vorgesehen sein, dass Anhand der Abnutzung und/oder der Abnutzungsfaktoren eine Vorhersage bezüglich der Abnutzung einzelner Komponenten und/oder des Beatmungsgerätes getroffen wird. Beispielsweise wird die Abnutzung dabei extrapoliert. Bei der Berechnung der Vorhersage kann beispielsweise auch der Verlauf der Abnutzung miteinbezogen werden. Beispielsweise wird der jüngere zeitliche Verlauf der Abnutzung stärker gewichtet als der zeitlich weiter zurückliegende. In manchen Ausführungsformen werden zusätzlich oder alternativ die Abnutzungsfaktoren und/oder der Gesamtfaktor als Grundlage der Vorhersage verwendet.

Anhand der Vorhersage der Abnutzung kann beispielsweise eine Zeitdauer berechnet werden, bis eine Wartung und/oder Austausch der Komponente und/oder des Beatmungsgerätes fällig wird. Es kann über die Vorhersage beispielsweise ein optimaler Wartungszeitpunkt ermittelt werden.

Alternativ oder ergänzend kann auch vorgesehen sein, dass die tatsächliche Abnutzung über verschiedene Parameter gemessen werden kann. Beispielsweise ist es denkbar, dass die Vibrationen erfasst werden, welche die Turbine/das Gebläse erzeugt, wobei beispielsweise auf den Zustand der Lager geschlossen werden kann. Auch kann die Wärmeleistung einzelner Komponenten, also wie stark sich die Komponenten während des Betriebs erhitzen und gegebenenfalls auch nach dem Betrieb wieder abkühlen, also Parameter erfasst werden, aus welchen Rückschlüsse über die Abnutzung getroffen werden können. Eine stärkere Erwärmung bei gleichbleibender Leistung einer Komponente kann etwa ein Hinweis auf eine fortschreitende Abnutzung sein. Auch ein pneumatischer Widerstand kann genutzt werden um beispielsweise die Verschmutzung einzelner Komponenten, insbesondere von Filtern, zu erfassen. Auch die Agilität des Beatmungsgerätes, also beispielsweise wie schnell ein Umschalten zwischen verschiedenen Drücken und/oder Flüssen erfolgt, kann Hinweise auf die Abnutzung einzelner Komponenten, beispielsweise Ventile und/oder die Atemgasquelle, geben.

Es kann auch vorgesehen sein, dass die Steuereinheit dazu eingerichtet und ausgebildet ist, einen Wartungszeitpunkt anhand der einzelnen Abnutzungen zu ermitteln, wobei eine möglichst große Zahl an Komponenten in ein Wartungsfenster fällt. Beispielsweise kann, insbesondere für nicht-kritische Komponenten, der Wartungszeitpunkt herausgezögert werden, sodass andere Komponenten ebenfalls in ein Wartungsfenster kommen. Dabei kann vorgesehen sein, dass eine bestimmte kritische Abnutzung nicht überschritten wird. Diese kritische Abnutzung löst beispielsweise eine sofortige Wartungsaufforderung aus.

Das Überschreiten eines Schwellwerts für die Gesamtabnutzung und/oder der Abnutzung einer einzelnen und/oder mehrerer Komponenten können eine oder mehrere Aktionen vorgesehen sein.

Eine Aktion kann beispielsweise die Erzeugung einer Mitteilung sein. Die Mitteilung kann beispielsweise vom Beatmungsgeräte erzeugt werden. In manchen Ausführungsformen ist vorgesehen, dass die Mitteilung an eine entfernte Gegenstelle übermittelt wird, beispielsweise eine Software, eine Cloud, eine App, einen Server und/oder andere externe Anzeigegeräte. Die Mitteilung kann dann beispielsweise einen Betreuer auf eine notwendige Wartung hinweisen und/oder über den Abnutzungsstatus des Beatmungsgerätes informieren.

In manchen Ausführungsformen ist vorgesehen, dass die Daten zur Ermittlung der Abnutzung und/oder der

EP 4 156 198 A1

Abnutzungsfaktoren an eine entfernte Gegenstelle übermittelt werden und die entfernte Gegenstelle die Abnutzung berechnet. Zudem kann vorgesehen sein, dass die entfernte Gegenstelle eine Mitteilung bezüglich der Abnutzung, etwa einen Hinweis auf eine notwendige Wartung und/oder über den Abnutzungsstatus des Beatmungsgerätes, erzeugt. Die Mitteilung kann an das Beatmungsgerät übermittelt und dort gegebenenfalls angezeigt werden. Es kann auch vorgesehen sein, dass aus der Mitteilung eine Nachricht, beispielsweise per Post/Brief und/oder E-Mail, versendet wird. Auch eine Anzeige der Mitteilung über eine App kann in manchen Ausführungsformen vorgesehen sein.

Die Mitteilung kann auch eine Information und/oder Daten dazu enthalten, in welcher Zeitdauer, beispielsweise basieren auf einer Vorhersage, eine Wartung und/oder Austausch von Komponenten und/oder des Beatmungsgerätes nötig wird.

Für manche Ausführungsformen ist vorgesehen, dass die Mitteilung Hinweise darauf enthält, wann und/oder das eine Wartung nötig ist. Die Hinweise können beispielsweise in verschiedenen Eskalationsstufen gegeben werden und gegebenenfalls mit weiteren Aktionen verknüpft werden. Ist beispielsweise in einem zukünftigen Zeitraum eine Wartung vorgesehen, etwa basierend auf einer Vorhersage, kann eine Meldung auf dem Beatmungsgerät angezeigt werden, welche zu einer Wartung auffordert. Es kann dabei vorgesehen sein, dass der Benutzer die Meldung bestätigen muss.

Ist der Zeitpunkt erreicht, bei dem die Abnutzung einen Schwellenwert erreicht und/oder überschreitet, bei dem eine Wartung nötig ist, kann eine nächste Eskalationsstufe erreicht werden. In dieser Stufe kann der Benutzer beispielsweise bei jeder weiteren Nutzung und/oder in periodischen, kürzer werdenden Zeitabständen, darauf hingewiesen werden, dass eine Wartung und/oder Komponententausch nötig ist. Vor der Nutzung des Beatmungsgerätes kann beispielsweise vorgesehen sein, dass diese Meldung durch den Benutzer bestätigt werden muss. Es kann beispielsweise auch vorgesehen sein, dass zusätzlich eine Weiterleitung angeboten wird und/oder eine Kontaktinformation eingeblendet wird, über welche weitere Schritte der Wartung erklärt oder vereinbart werden können. Beispielsweise kann zu einer Terminbuchung für eine Wartung weitergeleitet werden. In manchen Ausführungsformen wird bei erfolgreicher Terminbuchung eine Rückmeldung an das Beatmungsgerät gegeben, welche die Hinweise zur Wartung zumindest vorrübergehend deaktiviert. Im Falle einer Terminbuchung kann beispielsweise auch vorgesehen sein, dass das Beatmungsgerät eine Erinnerung generiert, welche den Benutzer auf einen bevorstehenden Wartungstermin hinweist. Alternativ oder ergänzend kann vorgesehen sein, dass eine entsprechende Meldung per Post und/oder E-Mail und/oder App erfolgt. Es kann zum Beispiel vorgesehen sein, dass eine Terminbestätigung per Post versandt wird und/oder eine Terminerinnerung per E-Mail und/oder App dem Benutzer des Beatmungsgerätes übermittelt wird. In manchen Ausführungsformen wird der Übermittlungsweg davon abhängig gemacht, welche Wege bei dem jeweiligen Benutzer zur Verfügung stehen. Wird beispielsweise keine App genutzt und/oder ist keine E-Mail-Adresse hinterlegt, kann vorgesehen sein, dass Mitteilungen bzw. Meldungen ausschließlich über Post und/oder das Beatmungsgerät übermittelt werden.

Alternativ oder ergänzend kann vorgesehen sein, dass gleichzeitig eine Meldung über eine externe Gegenstelle angezeigt wird, beispielsweise für einen Betreuer, dass das Beatmungsgerät und/oder eine Komponente gewartet und/oder getauscht werden muss. Sind beispielsweise Schritte zur Wartung und/oder zum Austausch eingeleitet, kann vorgesehen sein, dass über eine Rückmeldung an das Beatmungsgerät die Hinweise zur Wartung zumindest vorrübergehend deaktiviert werden.

Eine weitere Eskalationsstufe kann beispielsweise erreicht werden, wenn ein bestimmter Zeitraum überschritten wird, nachdem der Hinweis zur nötigen Wartung generiert wurde. Es kann beispielsweise vorgesehen sein, dass das Beatmungsgerät einen Hinweis bzw. eine Meldung generiert und/oder anzeigt, welcher zur sofortigen Wartung auffordert und sich nicht ausblenden lässt. In manchen Ausführungsformen kann auch vorgesehen sein, dass das Beatmungsgeräte und/oder einzelne Funktionen gesperrt werden, wenn die Wartung über einen Zeitraum ausbleibt. Das Sperren des Beatmungsgerätes bzw. einzelner Funktionen kann auch als weitere Eskalationsstufe vorgesehen sein.

Alternativ oder ergänzend kann vorgesehen sein, dass bestimmte Komponenten in einen Schonbetrieb übergehen. Im Schonbetrieb werden die betroffenen Komponenten dann beispielsweise mit geringerer Leistung betrieben. Beispielsweise wird die Heizleistung des Atemgasbefeuchters verringert, was zu einer längeren Lebensdauer bei einer geringeren Leistung führt. Auch kann beispielsweise die Turbine/das Gebläse mit einer geringeren Leistung betrieben werden. So kann die Lebensdauer, somit auch die Abnutzung, verlängert werden. Beispielsweise kann vorgesehen sein, bei ausbleibender Wartung die Leistung stufenweise zu reduzieren. Dabei kann auch vorgesehen sein, dass in bestimmten Situationen der Schonbetrieb ausgesetzt wird um in Bedarfsfällen eine hohe Leistung abrufen zu können. Dies betrifft insbesondere Situationen bei denen die Sicherheit und/oder Gesundheit des Patienten beeinträchtigt werden kann. Fällt der Patient etwa in eine Atemsituation bzw. Atemproblem, welche eine hohe Leistung bestimmter Komponenten erfordert, können diese zum Beheben der Atemprobleme aus dem Schonbetrieb genommen werden.

Der Schonbetrieb kann beispielsweise auch für verknüpfte Komponenten gelten. Bedarf der Akkumulator beispielsweise eine Wartung und wird in den Schonbetrieb überführt, so kann damit zusammenhängend beispielsweise auch die Leistung der Atemgasquelle verringert werden um den Akkumulator zusätzlich zu schonen.

Sollte durch fehlende Wartung des Beatmungsgerätes und/oder einzelner Komponenten die Therapie des Patienten unmöglich werden, kann vorgesehen sein, dass ein Alarm generiert wird. Dieser Alarm kann beispielsweise auf dem Beatmungsgerät ausgegeben werden um den Patienten zu informieren. Auch eine Alarmmeldung an einer entfernten Gegenstelle, beispielsweise beim Betreuer und/oder Provider, kann vorgesehen sein. In manchen Ausführungsformen kann vorgesehen werden, dass mit der Alarmmeldung der Versand eines Ersatzgerätes einhergeht.

Das Beatmungsgeräte bzw. die Steuereinheit kann beispielsweise auch dazu eingerichtet und ausgebildet sein, eine Wartung und/oder den Tausch von Komponenten zu erkennen und die Abnutzung daraufhin automatisch zurücksetzen. Beispielsweise kann die Abnutzung der gewarteten und/oder getauschten Komponenten zurückgesetzt werden. Wurde das Beatmungsgerät aufgrund einer ausbleibenden Wartung gesperrt, kann vorgesehen sein, dass durch die erfolgte Wartung das Beatmungsgeräte automatisch entsperrt wird. Alternativ oder ergänzend kann vorgesehen sein, dass nach erfolgter Wartung das Beatmungsgerät manuell entsperrt werden muss, beispielsweise durch einen Betreuer.

In manchen Ausführungsformen können bestimmte Wartungsarbeiten als Self-Service durch den Benutzer bzw. den Patienten selbst durchgeführt werden. Ist beispielsweise für Komponenten, welche durch den Patienten gewartet und/oder ausgetauscht werden können eine Abnutzung erreicht, welche eine Wartung und/oder einen Austausch vorsieht, kann zusätzlich zu der Meldung, dass eine Wartung vorgesehen ist, auch eine Information wiedergegeben werden, welche die Wartungsschritte aufzeigt. In manchen Ausführungsformen ist auch vorgesehen, dass für die Wartung von Komponenten, welche der Patient selbstständig durchführen kann, die benötigten Teile, z.B. Ersatzkomponenten, an den Patienten automatische versandt werden, sobald die Wartung als notwendig erkannt wird. Das Beatmungsgerät kann beispielsweise dazu eingerichtet sein, eine erfolgte Wartung zu erkennen und die Abnutzung zurückzusetzen. Auch etwaige Hinweise und Aufforderungen zur Wartung können dann automatisch durch das Beatmungsgerät deaktiviert werden.

Alternativ oder ergänzend kann beim Erreichen des Schwellwertes zur Wartung/zum Austausch automatisch ein Versand von Wartungsmaterial und/oder Austauschkomponenten an einen zuvor definierten Ort angestoßen werden. Beispielsweise wird der Versand einer Austauschkomponente automatisch so angestoßen, dass diese zusammen mit einem Wartungstermin an dem Ort der Wartung eintrifft.

Für bestimmte Komponenten kann auch vorgesehen sein, dass neben einer Abnutzung auch ein Zähler eingerichtet wird, welcher den Status bis zum Austausch der Komponente wiedergibt. Beispielsweise kann durch eine Wartung, und damit Zurücksetzen der Abnutzung der Komponenten auch der Zähler verringert werden, wobei der Zähler nur durch Austausch komplett zurückgesetzt werden kann. Dies betrifft beispielsweise Komponenten, deren Lebenszeit durch eine regelmäßige Wartung verlängert werden kann.

Nach erfolgter Wartung wird in manchen Ausführungsformen der Wartungszeitpunkt im Beatmungsgerät hinterlegt. In manchen Ausführungsformen ist die Steuereinheit dazu eingerichtet und ausgebildet, anhand der Wartungszeitpunkte eine zusätzliche oder alternative Abschätzung bzw. Vorhersage bezüglich zukünftiger Wartungszeitpunkte zu generieren.

In manchen Ausführungsformen werden die Abnutzungsfaktoren und/oder die Teilfaktoren über eine künstliche Intelligenz angepasst. Es kann dazu vorgesehen sein, dass die Effektivität, etc. einer Komponente immer wieder geprüft und/oder aufgezeichnet wird. Über die künstliche Intelligenz wird beispielsweise der Abnutzungsstatus mit der Leistung der jeweiligen Komponente verglichen, wobei der Abnutzungsfaktor gegebenenfalls entsprechend korrigiert. Nutzt sich beispielsweise der Turbinenmotor schneller ab, als über den Abnutzungsfaktor vorhergesagt, kann die künstliche Intelligenz den Abnutzungsfaktor des Turbinenmotors bzw. die einbezogenen Teilfaktoren anpassen um im weiteren Verlauf eine genauere Vorhersage zu treffen. Die Abnutzung des Turbinenmotors kann beispielsweise darüber erkannt werden, dass mehr Energie für die gleiche Drehzahl benötigt wird.

Für manche Ausführungsformen ist vorgesehen, dass anhand der Abnutzung eine Gebühr ermittelt wird. Beispielsweise erzeugt die Steuereinheit einen periodischen Bericht, welcher die Abnutzung und/oder eine auf der Abnutzung basierende Gebühr enthält. In manchen Ausführungsformen kann dieser Bericht automatisch als Rechnung an einen vordefinierten Empfänger gesendet werden. Es ist beispielsweise vorgesehen, dass eine Gesamtgebühr pro Zeitintervall, beispielsweise pro Woche/Monat/Quartal/Jahr, aus einer Basisgebühr und einer Gebühr basierend auf der ermittelten Abnutzung, z.B. eine Abnutzungsgebühr, einzelner Komponenten und/oder der Gesamtabnutzung errechnet wird. Beispielsweise ergibt sich eine Summe

Gesamtgebühr pro Zeitintervall = Basisgebühr + (Nutzungszeit im Zeitintervall * Abnutzungsfaktor) * Gebührenfaktor,

wobei das Produkt aus Nutzungszeit und Abnutzungsfaktor je Komponente einzeln berechnet wird. Es kann auch vorgesehen sein, dass zusätzlich ein Gewichtungsfaktor einbezogen wird. Dieser Gewichtungsfaktor kann beispielsweise auch miteinbeziehen in wie weit die jeweilige Abnutzung abhängig vom Patienten und/oder Therapie ist und in wie weit eine nicht zu vermeidende Abnutzung entsteht. Wir das Beatmungsgerät beispielsweise in einem Zeitintervall nicht genutzt, so fällt etwa nur die Basisgebühr für dieses Zeitintervall an.

Auch ist vorgesehen, dass die Abnutzungsgebühr automatisch an die Abnutzung angepasst wird. In manchen Ausführungsformen kann auch vorgesehen sein, einen Mittelwert, Median und/oder Trend der Abnutzungsgebühren zu bestimmen und für künftige Zeitintervalle zu Grunde zu legen, etwa als Abschlagszahlung.

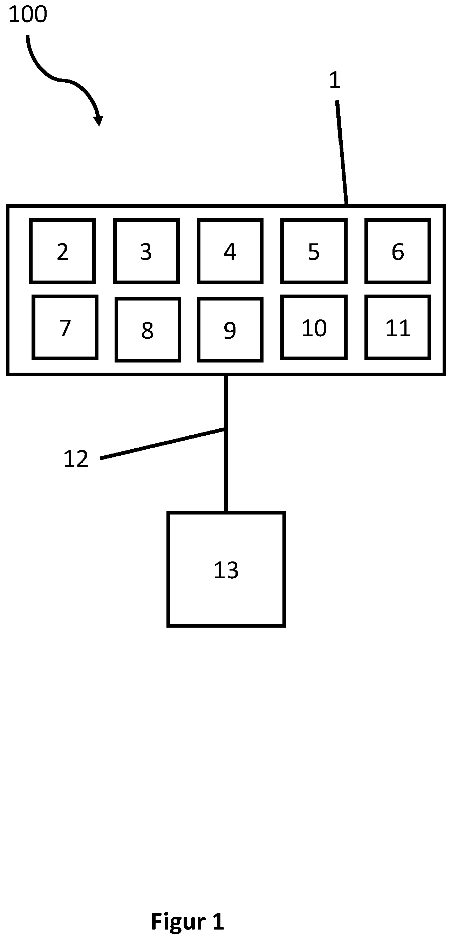

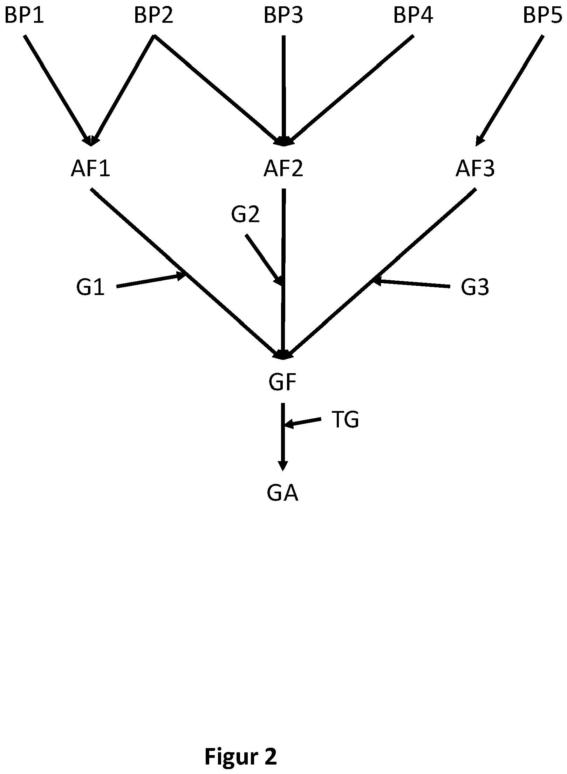

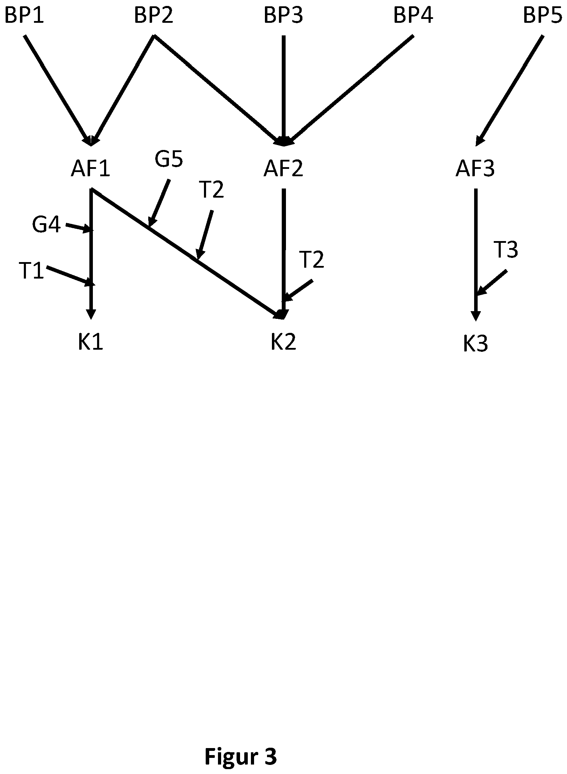

**Figur 1** zeigt eine beispielhafte Ausführungsform der Vorrichtung 100 zur Bestimmung der Nutzungsintensität eines Beatmungsgerätes 1. Figuren 2 und 3 zeigen in einer stark vereinfachten Weise beispielhaft die Ermittlung der Abnutzungsfaktoren und des Gesamtfaktors.

Das Beatmungsgerät 1 umfasst beispielhaft eine Steuereinheit 2, eine Nutzungsuhr 3 eine Sensoreinheit 4, ein Atemgasquelle 5, eine Erkennungseinheit 6, eine Eingabeeinheit 7, eine Anzeigeeinheit 8, eine Schnittstelle 9, einen Befeuchter 10 und eine Sauerstoffquelle 11. Mit dem Beatmungsgerät 1 ist über ein Schlauchsystem 13 ein Patienteninterface 14 verbunden.

Die Nutzungsuhr 3 ist so eingerichtet und ausgebildet, dass sie die Zeitdauer der Nutzung des Beatmungsgerätes 1 erfassen kann. In manchen Ausführungsformen ist die Nutzungsuhr 3 auch dazu ausgebildet, eine separate Zeitdauer für die Nutzung einzelner Komponenten und/oder Funktionen des Beatmungsgerätes 1 zu erfassen. In manchen Ausführungsformen ist die Nutzungsuhr 3 so eingerichtet und ausgebildet, dass die Zeitdauer der Nutzung nur während eines Zeitintervalls bzw. einer Zeiteinheit, zum Beispiel pro Tag, erfasst wird und danach beispielsweise an eine entfernte Gegenstelle übermittelt wird, welche die Zeitdauer der Nutzung dann über mehrere Zeiteinheiten/Zeitintervalle aufaddiert. Auch dabei kann es vorgesehen sein, dass ausschließlich oder zusätzlich die Zeitdauer der Nutzung einzelner Komponenten erfasst wird.

Die Steuereinheit 2 ist dazu eingerichtet und ausgebildet, die Abnutzung einzelner Komponenten und/oder des gesamten Beatmungsgerätes 1 zu bestimmen. Die Steuereinheit verrechnet dazu zumindest einen Abnutzungsfaktor mit der von der Nutzungsuhr 3 erfassten Zeitdauer der Nutzung. Die Steuereinheit 2 ist zudem dazu eingerichtet und ausgebildet, Abnutzungsfaktoren zu ermitteln. Die Abnutzungsfaktoren werden über die Steuereinheit 2 beispielsweise anhand verschiedener Betriebsparameter ermittelt. Es kann vorgesehen sein, dass einzelnen Komponenten separate Abnutzungsfaktoren zugeordnet werden, auch um separate Abnutzungen der jeweiligen Komponente zu bestimmen. Mehrere Abnutzungsfaktoren können zum Beispiel zu einem Gesamtfaktor verrechnet bzw. kombiniert werden, welcher dazu genutzt wird eine Gesamtabnutzung für das Beatmungsgerät 1 zu ermitteln. Wird nur ein Abnutzungsfaktor ermittelt, so kann dieser dem Gesamtfaktor entsprechen. In manchen Ausführungsformen wird entsprechend auch nur eine Abnutzung für das Beatmungsgerät 1 ermittelt, wobei diese Abnutzung dann beispielsweise die Gesamtabnutzung darstellt.

Betriebsparameter, welche die Grundlage der Abnutzungsfaktoren bilden können beispielsweise sein: Firmware-Version; Hardware-Version; Therapiemodus; Therapiedrücke; IPAP/EPAP-Rampe; Leckage; Drehzahl der Turbine/Gebläse; Drehzahländerungen der Turbine; Bremsvorgänge der Turbine; Schaltvorgänge von Ventilen; Trigger-Einstellungen; Atemfluss; Atemfrequenz; verwendete Maske; verwendetes Schlauchsystem; Anfeuchter; Umgebungstemperatur; Umgebungsluftfeuchtigkeit; Anzahl der hygienischen Aufbereitungen; Tag- oder Nachtbetrieb; Sauerstoffeinspeisung; Dauer und/oder Menge der Sauerstoffzumischung; Verwendung von Zusatzfunktionen wie Schlauchheizung und/oder Atemgasbefeuchtung; Häufigkeit von Beatmungsmanövern; Nutzung von zusätzlichen Sensoren; Status und/oder Quelle der Energieversorgung; Zeitpunkt der letzten Wartung. Die Steuereinheit 2 ist dazu eingerichtet, die Betriebsparameter zu registrieren bzw. zu erfassen und in Abnutzungsfaktoren einzubeziehen bzw. zu verrechnen. In manchen Ausführungsformen erfassen einzelne Komponenten die Betriebsparameter und übermitteln diese an die Steuereinheit 2, welche dazu eingerichtet ist die übermittelten Betriebsparameter in die jeweiligen Abnutzungsfaktoren einzubeziehen bzw. zu verrechnen.

In manchen Ausführungsformen umfasst die Vorrichtung 100 zumindest eine Speichereinheit (nicht dargestellt), welche dazu eingerichtet ist Betriebsparameter, Abnutzungsfaktoren, Abnutzungen und/oder Zeitdauern der Nutzung zu speichern und für einen Abruf bereitzustellen. Beispielsweise werden die von einzelnen Komponenten ermittelte Betriebsparameter in der Speichereinheit gespeichert, sodass die Steuereinheit 2 darauf zugreifen kann und die nötigen Daten/Werte/Informationen der Betriebsparameter abrufen kann.

Es ist vorgesehen, dass zumindest ein Betriebsparameter durch die Steuereinheit 2 in einen Abnutzungsfaktor einbezogen wird. Es kann zudem vorgesehen sein, dass unterschiedliche Abnutzungsfaktoren auch unterschiedliche Betriebsparameter berücksichtigen und/oder die Betriebsparameter unterschiedlich gewichtet werden.

Die Sensoreinheit 4 ist dazu eingerichtet und ausgebildet Drücke, Flüsse, Temperaturen und/oder Feuchtigkeit zu erfassen und gegebenenfalls auszuwerten. Beispielsweise ist die Sensoreinheit dazu mit Sensoren verbunden, welche im Beatmungsgerät 1 angeordnet sein können und/oder extern mit dem Beatmungsgerät 1 verbunden sind. Beispielsweise ist die Sensoreinheit 4 dazu eingerichtet und ausgebildet, aus einem Atemgasdruck und/oder Atemgasfluss Eigenschaften wie einen Atemfluss, ein Atemzugsvolumen, eine Atemfrequenz, Phasen der Atmung (zumindest Inspiration und Exspiration) zu bestimmen. Die von der Sensoreinheit erfassten Werte und Daten können beispielsweise als Betriebsparameter in Abnutzungsfaktoren einbezogen werden oder als Grundlage der Bestimmung von Betriebsparametern dienen. In manchen Ausführungsformen ist die Sensoreinheit 4 auch dazu ausgebildet und eingerichtet Umgebungstemperaturen und/oder den Umgebungsdruck zu erfassen.

Die steuerbare Atemgasquelle 5 ist dazu eingerichtet und ausgebildet, Atemgas zu und/oder von einem Patienten zu Fördern. Beispielsweise kann die Atemgasquelle 5 als Gebläse ausgebildet sein. Das Gebläse umfasst beispielsweise eine Turbine mit Turbinenmotor und Turbinenrad. Beispielsweise ist das Gebläse dazu eingerichtet und ausgebildet eine Drehzahl des Motors bzw. des Turbinenrads zu erfassen. Die erfasste Drehzahl kann als Betriebsparameter in Abnutzungsfaktoren einbezogen werden und dazu direkt an die Steuereinheit 2 übermittelt werden und/oder in der Speichereinheit (zwischen-)gespeichert werden.

In manchen Ausführungsformen ist die Steuereinheit 2 dazu eingerichtet, das Gebläse anzusteuern, beispielsweise um einen vorgegebenen Druck und/oder Fluss zu erzeugen. In manchen Ausführungsformen wird dazu eine Drehzahl des Motors vorgegeben. Es kann auch vorgesehen sein, dass die Steuereinheit 2 einen Druck und/oder Fluss vorgibt und da Gebläse so ansteuert, dass dieser Druck und/oder Fluss erreicht wird. Es kann auch vorgesehen sein, dass die Steuereinheit 2 und/oder das Gebläse und/oder eine Erkennungseinheit 6 den Energieeintrag zum Motor bzw. zum Gebläse ermittelt. Beispielsweise kann anhand des Energieeintrags in Relation zu der erreichten Drehzahl des Motors und/oder zum erreichten Druck bzw. Fluss ein Betriebsparameter und/oder ein Abnutzungsfaktor ermittelt werden.

Ferner kann vorgesehen sein, dass auch eine Relation zwischen erreichtem Druck bzw. Fluss und der Drehzahl des Motors mit in Betriebsparameter bzw. Abnutzungsfaktoren einfließt bzw. verrechnet wird.

In manchen Ausführungsformen ist die Atemgasquelle 5 alternativ oder ergänzend als Druckgasanschluss ausgeführt, welcher mit einer externen Druckgasquelle verbunden ist. Beispielsweise über Ventile kann der Atemgasfluss und Atemgasdruck reguliert werden. Es kann vorgesehen sein, dass die Steuereinheit 2 und/oder eine Zähleinheit eine Anzahl an Ventilschaltungen aufzeichnet und diese als Betriebsparameter für Abnutzungsfaktoren zugrunde legt. In manchen Ausführungsformen kann vorgesehen sein, dass neben der Anzahl der Schaltungen auch eine Zeit zwischen den Schaltvorgängen registriert wird. Besonders schnelle Schaltungen, bei den die Zeit zwischen den Schaltvorgängen einen Schwellenwert unterschreiten, können beispielsweise mit einem höheren Faktor in die Abnutzungsfaktoren eingehen.

Die Erkennungseinheit 6 ist beispielhaft dazu ausgebildet, verschiedene Betriebsparameter zu erfassen, welche in die Abnutzungsfaktoren miteinbezogen werden können. Beispielsweise kann vorgesehen sein, dass die Erkennungseinheit 6 dazu eingerichtet ist, angeschlossenes Zubehör und/oder Komponenten zu erkennen. Es kann zum Beispiel vorgesehen sein, dass manche Komponenten eine Typenbezeichnung, Seriennummer und/oder Versionsinformation an die Erkennungseinheit 6 übermitteln, wobei die Erkennungseinheit 6 die Komponente anhand dieser Informationen erkennt. Es kann dazu vorgesehen sein, dass die Komponenten dazu elektrisch mit einem Mainboard oder einer Hauptplatine verbunden werden und die entsprechenden Informationen zum Abruf bereitstellen. Es kann vorgesehen sein, dass die Erkennungseinheit 6 und/oder die Steuereinheit 2 anhand der erkannten Komponente, beispielsweise unter Einbezug einer Versionsinformation, die Abnutzungsfaktoren anpasst.

In manchen Ausführungsformen ist die Steuereinheit 2, beispielsweise in Kombination mit der Erkennungseinheit 6, dazu ausgebildet eine erfolgte Wartung des Beatmungsgerätes 1 und/oder einzelner Komponenten automatisch zu erfassen. Dies kann beispielsweise anhand der Veränderung verschiedener Betriebsparameter erfolgen. Wurde etwa der Motor der Turbine gewartet, kann etwa die Steuereinheit 2 feststellen, dass nach der Wartung ein geringerer Energieeintrag für die gleiche Leistung nötig ist und daraus auf eine Wartung des Motors schließen. Im Falle eines Filters kann beispielsweise ein geringerer Gasdruck bereits ausreichen um den gleichen Fluss zu erzeugen wie vor der Wartung. Auch hier kann die Steuereinheit 2 auf einen Austausch oder die Wartung des Filters schließen.

Dadurch, dass die Erkennungseinheit 6 und/oder die Steuereinheit 2 die verbauten Komponenten erkennen, kann auch vorgesehen sein, dass ein Austausch verschiedener Komponenten erkannt wird. Ist beispielsweise eine Wartung und/oder ein Austausch einer Komponente, beispielsweise aufgrund der ermittelten Abnutzung, nötig, kann die Steuereinheit 2 feststellen, ob die Wartung oder der Austausch durchgeführt wurde.

Ergänzend oder alternativ ist die Erkennungseinheit 6 dazu eingerichtet und ausgebildet, Betriebsparameter wie Energieverbrauch, Geräteleistung, Energiequelle und/oder kritische Fehler zu erfassen. Kritische Fehler können beispielsweise der Ausfall von verschiedenen Komponenten und/oder Funktionen sein. Auch punktuelle Überlastungen - beispielsweise der Atemgasquelle - können durch die Erkennungseinheit 6 erfasst werden. Auch kann die Erkennungseinheit 6 dazu eingerichtet sein, Temperaturen, wie zum Beispiel eine Betriebstemperatur des Beatmungsgerätes und/oder einzelner Komponenten, zu messen und erfassen. So kann beispielsweise festgestellt werden, ob einzelne Komponenten und/oder das gesamte Beatmungsgerät 1 einer erhöhten Temperatur ausgesetzt sind.

Die Erkennungseinheit 6 ist ferner dazu eingerichtet, Peripheriegeräte/Zubehör wie ein angeschlossenes Patienteninterface, ein Schlauchsystem und/oder einen angeschlossenen Befeuchter zu erkennen. Ferne kann vorgesehen sein, dass die Erkennungseinheit 6 auch erkennt, um welches Patienteninterface und/oder Schlauchsystem und/oder Befeuchter es sich handelt. Eine solche Erkennung kann zum Beispiel über RFID-Chips in den Komponenten verwirklicht werden, welche von der Erkennungseinheit 6 automatisch erfasst werden können. Es kann auch vorgesehen sein, dass über eine Schnittstelle automatisch bei Anschluss Informationen zu Typ, Version, etc. des Zubehörs an die Erkennungseinheit 6 und/oder die Steuereinheit 2 übermittelt werden und so eine Erkennung des Zubehörs möglich ist. Über das erkannte Zubehör kann die Steuereinheit 2 dann gegebenenfalls auch Informationen zu Widerstand, Heizleistung, etc. erfassen.

In manchen Ausführungsformen kann vorgesehen sein, dass, sollte die Erkennungseinheit 6 oder die Steuereinheit 2 das angeschlossene Zubehör nicht erkennen, über Druck und Flusstests zumindest festgestellt werden kann, ob überhaupt ein Zubehör angeschlossen ist.

Die Eingabeeinheit 7 ist dazu eingerichtet, das Daten, Informationen und/oder Werte in das Beatmungsgerät 1 eingegeben werden können. Beispielsweise können über die Eingabeeinheit 7 Meldungen, Hinweise, Warnungen etc. bestätigt werden. Auch kann in manchen Ausführungsformen vorgesehen sein, dass eine erfolgte Wartung über die Eingabeeinheit 7 bestätigt und/oder erfasst wird. Die Eingabeeinheit 7 ist zudem dazu eingerichtet, dass Vorgaben zu der Atemtherapie gemacht werden können. So kann etwa ein Druck, Fluss, Therapiemodus, etc. über die Eingabeeinheit 7 festgelegt werden. Die Eingabeeinheit 7 kann zum Beispiel als Tastatur und/oder weiteren Bedienelemente wie Knöpfen und Drehreglern ausgebildet sein.

Über die Anzeigeeinheit 8, beispielsweise als Display ausgebildet, können verschiedene Daten, Informationen, Werte, Meldungen, Hinweise und/oder Alarme am Beatmungsgerät 1 ausgegeben werden. Eine Darstellung kann beispielsweise graphisch und/oder alphanumerisch erfolgen. Meldungen und Hinweise können unteranderem Informationen zur Abnutzung des Beatmungsgerätes und/oder einzelner Komponenten enthalten. Beispielsweise kann über eine bevorstehende Wartung informiert werden. Auch können kritische Meldungen, etwa bei einem Überschreiten eines Wartungszeitraumes oder Wartungszeitpunkts, angezeigt werden.

Es kann auch vorgesehen sein, dass die Anzeigeeinheit 8 und die Eingabeeinheit 7 alternativ oder ergänzend als eine gemeinsame Informations- und Bedienschnittstelle, etwa als berührungsempfindlicher Bildschirm (Touchscreen) ausgebildet ist.

Über die Schnittstelle 9 ist vorgesehen, dass eine Übermittlung von Meldungen und/oder Hinweisen, insbesondere zum Status des Beatmungsgerätes 1, an entfernte Gegenstellen ermöglicht wird. Der Status des Beatmungsgerätes 1 kann dabei unter anderem eine Gesamtabnutzung und/oder die Abnutzung einzelner Komponenten umfassen. Zudem kann vorgesehen sein, dass über die Schnittstelle 9 eine Meldung übermittelt wird, welche den Versand von zur Wartung nötigen Materialien und/oder Austauschkomponenten an eine vordefinierte Adresse ausgelöst werden. Die Auslösung kann beispielsweise automatisch erfolgen, sobald ein Wartungszeitpunkt erreicht wird. In manchen Ausführungsformen wird der Versand automatisch ausgelöst, wenn über das Beatmungsgerät 1 ein Wartungstermin, etwa als Folge einer nötigen Wartung, erstellt wird.

Der Befeuchter 10 ist beispielhaft dazu eingerichtet und ausgebildet, das Atemgas zu befeuchten und/oder zu erwärmen. Beispielsweise verfügt der Befeuchter 10 über eine Wasserkammer mit einem Heizelement. Über den Befeuchter 10 kann unter anderem die Feuchtigkeit des Atemgas eingestellt werden. In manchen Ausführungsformen ist die Steuereinheit 2 und/oder die Erkennungseinheit 6 dazu eingerichtet zu erkennen, um welchen Anfeuchter es sich handelt. Es kann auch vorgesehen sein, die Wassertemperatur und/oder die Atemgastemperatur messen zu können. Beispielsweise verfügt der Befeuchter 10 dazu über entsprechende Temperatursensoren. Die Temperatur des Wassers und/oder des Atemgases kann beispielsweise als Betriebsparameter in die Abnutzungsparameter einfließen. Es kann ferner vorgesehen sein, auch die gemessene und/oder zu erreichende Atemgasfeuchtigkeit als Betriebsparameter zu verwenden. In manchen Ausführungsformen kann vorgesehen sein, dass die Nutzungsuhr 3 eine Zeitdauer der Verwendung des Befeuchters 10 erfasst. Beispielsweise kann über die Zeitdauer der Verwendung des Befeuchters 10 zusammen mit einem entsprechenden Abnutzungsfaktor für den Befeuchter 10 eine Abnutzung für den Befeuchter 10 ermittelt werden.

Der optionale Sauerstoffzumischer 11 dient der Zumischung von Sauerstoff zum Atemgas. Der Sauerstoffzumischer 11 kann beispielsweise mit einer externen Sauerstoffquelle verbunden sein. Über den Sauerstoffzumischer 11 kann zum Beispiel die Menge und Dauer der Sauerstoffzumischung eingestellt werden.

Über das Schlauchsystem 12 ist das Patienteninterface 13 mit dem Beatmungsgerät 1 verbunden. Es kann vorgesehen sein, dass die Erkennungseinheit 6 und/oder die Steuereinheit 2 das Schlauchsystem 12 automatisch erkennt, zum Beispiel anhand eines RFID-Chips oder über einen Anschluss, über welchen das Schlauchsystem Informationen zu Typ, etc. übermittelt. In manchen Ausführungsformen umfasst das Schlauchsystem 12 eine Schlauchheizung. Die Dauer der Verwendung und Temperatur der Schlauchheizung kann dabei als Betriebsparameter von der Steuereinheit 2 in Abnutzungsfaktoren und/oder Abnutzungen verrechnet werden.

Zusammen mit dem Schlauchsystem 12 kann auch das Patienteninterface 13 automatisch von dem Beatmungsgerät 1 erkannt werden. Beispielsweise verfügt das Patienteninterface 13 über einen elektrischen Kontakt über welchen Informationen zu dem Patienteninterface 13 an das Beatmungsgerät 1 übertragen werden.

Die Steuereinheit 2 kann in manchen Ausführungsformen dazu eingerichtet und ausgebildet sein, über den Verlauf der Abnutzung und/oder der Abnutzungsfaktoren eine Vorhersage bezüglich der Abnutzung zu treffen. In manchen Ausführungsformen kann vorgesehen sein, dass die Steuereinheit 2 auch eine Vorhersage bezüglich eines Wartungstermins erzeugen kann. In manchen Ausführungsformen ist die Steuereinheit 2 dazu eingerichtet, jüngere Abnutzungsfaktoren stärker in eine Vorhersage einzubeziehen als ältere und/oder die den jüngeren Verlauf der Abnutzung stärker zu berücksichtigen.

In **Figur 2** ist beispielhaft und stark vereinfacht dargestellt, wie aus den Betriebsparametern bzw. den zugehörigen Teilfaktoren BP1, BP2, BP3. BP4, BP5 die Abnutzungsfaktoren AF1, AF2, AF3 ermittelt werden und wie die Abnutzungsfaktoren AF1, AF2, AF3 in den Gesamtfaktor GF einfließen.

Der Betriebsparameter des Atemgasflusses wird beispielsweise über den Teilfaktor BP1 dargestellt. Der Betriebsparameter des gewählten Therapiemodus wird beispielsweise über den Teilfaktor BP2 dargestellt. Der Betriebsparameter der Drehzahl der Turbine wird beispielsweise über den Teilfaktor BP3 dargestellt. Der Betriebsparameter des zu erreichenden Atemgasdrucks wird beispielsweise über den Teilfaktor BP4 dargestellt. Der Betriebsparameter des pneumatischen Widerstandes wird beispielsweise über den Teilfaktor BP5 dargestellt.

In den Abnutzungsfaktor AF1 gehen beispielsweise die Teilfaktoren BP1 und BP2 ein. Eine Verrechnung kann beispielsweise über eine Multiplikation von BP1 und BP2 erfolgen. Der Abnutzungsfaktor AF2 basiert beispielsweise auf einer Kombination des Therapiemodus BP2, der Drehzahl der Turbine BP3 und des Atemgasdruckes BP4. Die Teilfaktoren BP2, BP3 und BP4 können beispielsweise über eine Aufsummierung zu dem Abnutzungsfaktor AF2 kombiniert werden. Der Abnutzungsfaktor AF3 ist beispielsweise nur vom pneumatischen Widerstand BP5 abhängig.

Über die Gewichtungsfaktoren G1, G2, G3 werden die Abnutzungsfaktoren AF1, AF2, AF3 zu dem Gesamtfaktor GF verrechnet. Der Gesamtfaktor GF stellt einen Abnutzungsfaktor für das gesamte Beatmungsgerät dar. Über eine Verrechnung, beispielsweise eine Multiplikation, des Gesamtfaktors GF mit der Zeitdauer der Nutzung TG des Beatmungsgerätes wird die Gesamtabnutzung GA bestimmt.

**Figur 3** zeigt beispielhaft die Ermittlung der Abnutzung K1, K2, K3 einzelner Komponenten. Die Abnutzungsfaktoren AF1, AF2, AF3 werden wie zuvor unter Figur 2 beschrieben bestimmt. Die Abnutzung K1 stellt beispielsweise die Abnutzung eines angeschlossenen Atemgasbefeuchters dar. Um die Abnutzung K1 des Atemgasbefeuchters zu bestimmen wird beispielsweise der Abnutzungsfaktor AF1, welcher den Atemgasfluss und den gewählten Therapiemodus miteinbezieht, mit einem Gewichtungsfaktor G4 und einer Zeitdauer T1 der Nutzung verrechnet. Die Zeitdauer T1 kann beispielsweise eine Nutzungszeitdauer des Atemgasbefeuchters sein.

Die Abnutzung K2 ist beispielhaft die Abnutzung der Atemgasquelle. Die Abnutzung K2 der Atemgasquelle wird beispielsweise über die Abnutzungsfaktoren AF1 und AF2 bestimmt. Der Abnutzungsfaktor geht beispielsweise ohne Gewichtungsfaktor nur über die Zeitdauer T2 der Nutzung der Atemgasquelle in die Abnutzung K2 der Atemgasquelle ein. Der Abnutzungsfaktor AF1 hingegen wird über einen Gewichtungsfaktor G5 einberechnet.

Die Abnutzung K3 ist beispielhaft die Abnutzung eine im Beatmungsgerät verbauten Filters. Die Abnutzung K3 des Filters wird direkt über den Abnutzungsfaktor AF3 und die Zeitdauer T3 der Nutzung des Filters ermittelt.

Bezugszeichenliste

| | |
|---|---|
| 1 | Beatmungsgerät |
| 2 | Steuereinheit |
| 3 | Nutzungsuhr |
| 4 | Sensoreinheit |
| 5 | Atemgasquelle |
| 6 | Erkennungseinheit |
| 7 | Eingabeeinheit |
| 8 | Ausgabeeinheit |
| 9 | Schnittstelle |
| 10 | Befeuchter |
| 11 | Sauerstoffzumischer |
| 12 | Schlauchsystem |
| 13 | Patienteninterface |
| AF1 | Abnutzungsfaktor 1 |
| AF2 | Abnutzungsfaktor 2 |
| AF3 | Abnutzungsfaktor 3 |
| BP1 | Teilfaktor Betriebsparameter 1 |
| BP2 | Teilfaktor Betriebsparameter 2 |
| BP3 | Teilfaktor Betriebsparameter 3 |
| BP4 | Teilfaktor Betriebsparameter 4 |
| BP5 | Teilfaktor Betriebsparameter 5 |
| G1 | Gewichtungsfaktor 1 |
| G2 | Gewichtungsfaktor 2 |

G3     Gewichtungsfaktor 3
G4     Gewichtungsfaktor 4
G5     Gewichtungsfaktor 5
GA    Gesamtabnutzung
GF    Gesamtfaktor
K1     Abnutzung Komponente 1
K2     Abnutzung Komponente 2
K3     Abnutzung Komponente 3
T1     Zeitdauer Abnutzungsfaktor 1
T2     Zeitdauer Abnutzungsfaktor 2
T3     Zeitdauer Abnutzungsfaktor 3
TG    Zeitdauer der Nutzung

**Patentansprüche**

**1.** Vorrichtung zur Bestimmung einer Nutzungsintensität eines Beatmungsgerätes, aufweisend zumindest eine Steuereinheit und eine Nutzungsuhr, die ausgebildet und eingerichtet ist, die Zeitdauer der Nutzung des Beatmungsgerätes zu erfassen, **dadurch gekennzeichnet, dass** die Steuereinheit dazu eingerichtet und ausgebildet ist, die Zeitdauer der Nutzung des Beatmungsgerätes mit zumindest einem Abnutzungsfaktor zu verrechnen, um daraus zumindest eine Abnutzung zu ermitteln.

**2.** Vorrichtung nach zumindest einem der vorhergehenden Ansprüche, **dadurch gekennzeichnet, dass** die Steuereinheit dazu eingerichtet und ausgebildet ist, eine Mehrzahl von Abnutzungsfaktoren zu ermitteln.

**3.** Vorrichtung nach zumindest einem der vorhergehenden Ansprüche, **dadurch gekennzeichnet, dass** die Steuereinheit dazu eingerichtet und ausgebildet ist, die Abnutzungsfaktoren zu einem Gesamtfaktor für das Beatmungsgerät zu verrechnen und über eine Verrechnung des Gesamtfaktors mit der Zeitdauer der Nutzung eine Gesamtabnutzung des Beatmungsgerätes zu ermitteln.

**4.** Vorrichtung nach zumindest einem der vorhergehenden Ansprüche, **dadurch gekennzeichnet, dass** die Steuereinheit dazu eingerichtet und ausgebildet ist, zu einzelnen Komponenten und/oder Komponentengruppen des Beatmungsgerätes jeweils zumindest einen Abnutzungsfaktor zu ermitteln.

**5.** Vorrichtung nach zumindest einem der vorhergehenden Ansprüche, **dadurch gekennzeichnet, dass** die Steuereinheit dazu eingerichtet und ausgebildet ist, die Zeitdauer der Nutzung des Beatmungsgerätes mit den jeweiligen Abnutzungsfaktoren der Komponenten und/oder Komponentengruppen zu verrechnen, um daraus eine Abnutzung der jeweiligen Komponente und/oder Komponentengruppe zu ermitteln.

**6.** Vorrichtung nach zumindest einem der vorhergehenden Ansprüche, **dadurch gekennzeichnet, dass** die Steuereinheit dazu eingerichtet und ausgebildet ist, den Abnutzungsfaktor aus zumindest einem Betriebsparameter des Beatmungsgerätes zu bestimmen, wobei der zumindest eine Betriebsparameter ausgewählt wird aus Firmware-Version; Hardware-Version; Therapiemodus; Therapiedrücke; IPAP/EPAP-Rampe; Leckage; Drehzahl der Turbine/Gebläse; Drehzahländerungen der Turbine; Bremsvorgänge der Turbine; Schaltvorgänge von Ventilen; Trigger-Einstellungen; Atemfluss; Atemfrequenz; verwendete Maske; verwendetes Schlauchsystem; Anfeuchter; Umgebungstemperatur; Umgebungsluftfeuchtigkeit; Anzahl der hygienischen Aufbereitungen; Tag- oder Nachtbetrieb; Sauerstoffeinspeisung; Dauer und/oder Menge der Sauerstoffzumischung; Verwendung von Zusatzfunktionen wie Schlauchheizung und/oder Atemgasbefeuchtung; Häufigkeit von Beatmungsmanövern; Nutzung von zusätzlichen Sensoren; Status und/oder Quelle der Energieversorgung; Zeitpunkt der letzten Wartung.

**7.** Vorrichtung nach zumindest einem der vorhergehenden Ansprüche, **dadurch gekennzeichnet, dass** die Steuereinheit dazu eingerichtet und ausgebildet ist, für zumindest eine Komponente ausgewählt aus Turbine; Befeuchter; Wasserkammer; Luftfilter; Schlauch; Patienteninterface; Schalldämmschäume; Elektronik; Netzteil; Akkumulator; Sensoren; Messzellen; Ventile eine Abnutzung zu ermitteln, welche unabhängig von der Gesamtabnutzung ist.

**8.** Vorrichtung nach zumindest einem der vorhergehenden Ansprüche, **dadurch gekennzeichnet, dass** die Steuereinheit dazu eingerichtet und ausgebildet ist die Zahl und/oder Dauer der Ladezyklen oder die Kapazität oder den Innenwiderstand des Akkumulators oder andere Indikatoren für die Nutzung des Akkumulators zu ermitteln und für die Ermittlung zumindest eines Abnutzungsfaktors zu berücksichtigen.

9. Vorrichtung nach zumindest einem der vorhergehenden Ansprüche, **dadurch gekennzeichnet, dass** die Nutzungsuhr dazu eingerichtet und ausgebildet ist, neben der Zeitdauer der Nutzung des Beatmungsgerätes auch eine Standby-Zeit des Beatmungsgerätes zu erfassen.

10. Vorrichtung nach zumindest einem der vorhergehenden Ansprüche, **dadurch gekennzeichnet, dass** die Steuereinheit dazu eingerichtet und ausgebildet ist, die Standby-Zeit des Beatmungsgerätes mit in die Gesamtabnutzung des Beatmungsgerätes zu verrechnen.

11. Vorrichtung nach zumindest einem der vorhergehenden Ansprüche, **dadurch gekennzeichnet, dass** die Steuereinheit dazu eingerichtet und ausgebildet ist, zu jedem Abnutzungsfaktor einen durchschnittlichen Abnutzungsfaktor zu ermitteln und anhand des jeweiligen durchschnittlichen Abnutzungsfaktors eine Vorhersage bezüglich der jeweiligen Abnutzung zu treffen.

12. Vorrichtung nach zumindest einem der vorhergehenden Ansprüche, **dadurch gekennzeichnet, dass** die Steuereinheit dazu eingerichtet und ausgebildet ist, den durchschnittlichen Abnutzungsfaktor aus dem zeitlichen Verlauf des jeweils zugrundeliegenden Abnutzungsfaktors zu ermitteln, wobei der durchschnittliche Abnutzungsfaktor eine zeitliche Gewichtung enthält.

13. Vorrichtung nach zumindest einem der vorhergehenden Ansprüche, **dadurch gekennzeichnet, dass** die Steuereinheit dazu eingerichtet und ausgebildet ist, Abnutzungsfaktoren, welche im zeitlichen Verlauf jünger sind, für den durchschnittlichen Abnutzungsfaktor stärker zu gewichten.

14. Vorrichtung nach zumindest einem der vorhergehenden Ansprüche, **dadurch gekennzeichnet, dass** die Steuereinheit dazu eingerichtet und ausgebildet ist, anhand der ermittelten Abnutzung und/oder der Vorhersage zur Abnutzung eine Meldung zu generieren, welche zumindest einen Hinweis auf eine Wartung enthält.

15. Vorrichtung nach zumindest einem der vorhergehenden Ansprüche, **dadurch gekennzeichnet, dass** die Steuereinheit dazu eingerichtet und ausgebildet ist, zu erkennen, wenn die Wartung abgeschlossen ist, wobei die Meldung gelöscht wird und der Wartungszeitpunkt im Beatmungsgerät hinterlegt wird.

16. Vorrichtung nach zumindest einem der vorhergehenden Ansprüche, **dadurch gekennzeichnet, dass** die Steuereinheit dazu eingerichtet und ausgebildet ist, anhand der ermittelten Abnutzung und/oder der Vorhersage zur Abnutzung einen optimalen Wartungszeitpunkt ermittelt und ein entsprechende Meldung generiert.

17. Vorrichtung nach zumindest einem der vorhergehenden Ansprüche, **dadurch gekennzeichnet, dass** die Steuereinheit dazu eingerichtet und ausgebildet ist, nach einem Zeitraum, nachdem der Wartungszeitpunkt überschritten wird, eine Alarmmeldung zu genieren und bei weiterem Ausbleiben der Wartung das Beatmungsgerät und/oder bestimmte Funktionen des Beatmungsgerätes sperrt bis die Wartung durchgeführt wurde.

18. Vorrichtung nach zumindest einem der vorhergehenden Ansprüche, **dadurch gekennzeichnet, dass** die Steuereinheit dazu eingerichtet und ausgebildet ist, nach erfolgter Wartung das gesperrte Beatmungsgerät bzw. die gesperrten Funktionen wieder zu entsperren und/oder die Alarmmeldung zu deaktivieren.

19. Vorrichtung nach zumindest einem der vorhergehenden Ansprüche, **dadurch gekennzeichnet, dass** die Steuereinheit dazu eingerichtet und ausgebildet ist, anhand der ermittelten Abnutzung zu erkennen, ob eine Komponente des Beatmungsgerätes ausgetauscht werden muss und eine Austauschmeldung generiert, wobei die Austauschmeldung zumindest an eine entfernte Gegenstelle übermittelt wird und die Austauschmeldung an der entfernten Gegenstelle eine Aktion auslöst, welche zum Versand einer Ersatzkomponente an einen vordefinierten Ort führt.

20. Vorrichtung nach zumindest einem der vorhergehenden Ansprüche, **dadurch gekennzeichnet, dass** die Steuereinheit dazu eingerichtet und ausgebildet ist, zusammen mit der Austauschmeldung eine Information generiert, wie die auszutauschende Komponente zu wechseln ist und automatisch erkennt, wenn die auszutauschende Komponente durch eine neue Komponente ersetzt wird.

21. Vorrichtung nach zumindest einem der vorhergehenden Ansprüche, **dadurch gekennzeichnet, dass** die Steuereinheit dazu eingerichtet und ausgebildet ist, in periodischen Zeitabständen einen Nutzungsbericht zu erstellen, welcher zumindest eine Abnutzung umfasst und/oder eine auf der Abnutzung basierende Gebühr aufweist.

22. Vorrichtung nach zumindest einem der vorhergehenden Ansprüche, **dadurch gekennzeichnet, dass** die Steuereinheit dazu eingerichtet und ausgebildet ist, die Gebühr aus einer Basisgebühr und einer Abnutzungsgebühr zu berechnen, wobei die Abnutzungsgebühr automatisch an die Abnutzung angepasst wird.

23. Vorrichtung nach zumindest einem der vorhergehenden Ansprüche, **dadurch gekennzeichnet, dass** die Steuereinheit dazu eingerichtet und ausgebildet ist, einen Verbrauch an Datenvolumen als Betriebsparameter zu nutzen.

24. Vorrichtung nach zumindest einem der vorhergehenden Ansprüche, **dadurch gekennzeichnet, dass** die Steuereinheit dazu ausgebildet und eingerichtet ist, nach dem Verbrauch eines bestimmten Datenvolumens die gesendete Datenmenge und/oder die Frequenz der Datenübertragungen verringert wird und/oder eine manuelle Sendefunktion deaktiviert wird.

25. Verfahren zur Bestimmung einer Nutzungsintensität eines Beatmungsgerätes, wobei die Zeitdauer der Nutzung des Beatmungsgerätes erfasst wird, **dadurch gekennzeichnet, dass** die Zeitdauer der Nutzung des Beatmungsgerätes mit zumindest einem Gewichtungsfaktor verrechnet wird, um daraus eine Abnutzung zu ermitteln.

100

1

| 2 | 3 | 4 | 5 | 6 |
| 7 | 8 | 9 | 10 | 11 |

12

13

**Figur 1**

**Figur 2**

BP1     BP2     BP3     BP4     BP5

AF1     G5      AF2             AF3

G4      T2

T1      T2      T2      T3

K1      K2      K3

**Figur 3**

Europäisches
Patentamt

European
Patent Office

Office européen
des brevets

**EUROPÄISCHER RECHERCHENBERICHT**

Nummer der Anmeldung

EP 22 19 5828

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2011/259333 A1 (SANCHEZ GABRIEL [US] ET AL) 27. Oktober 2011 (2011-10-27) * Zusammenfassung * * Absatz [0081] – Absatz [0119] * ----- | 1-25 | INV. G16H20/40 G16H40/40 |
| X | WO 2020/092701 A2 (RESMED INC [US]; RESMED SENSOR TECH LTD [IE]) 7. Mai 2020 (2020-05-07) * Absatz [0203] – Absatz [0226] * ----- | 1-25 | |
| A | UMBELINO VANDA ET AL: "Standards about Medical Equipment Maintenance – A Survey", 2019 IEEE 6TH PORTUGUESE MEETING ON BIOENGINEERING (ENBENG), IEEE, 22. Februar 2019 (2019-02-22), Seiten 1-4, XP033559280, DOI: 10.1109/ENBENG.2019.8692460 [gefunden am 2019-04-15] * Seite 1, Spalte 2 – Seite 4, Spalte 2 * ----- | 1-25 | |

**RECHERCHIERTE SACHGEBIETE (IPC)**

G16H

Der vorliegende Recherchenbericht wurde für alle Patentansprüche erstellt

| Recherchenort | Abschlußdatum der Recherche | Prüfer |
|---|---|---|
| München | 6. Februar 2023 | Menschner, Philipp |

KATEGORIE DER GENANNTEN DOKUMENTE

X : von besonderer Bedeutung allein betrachtet
Y : von besonderer Bedeutung in Verbindung mit einer
 anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
 nach dem Anmeldedatum veröffentlicht worden ist
D : in der Anmeldung angeführtes Dokument
L : aus anderen Gründen angeführtes Dokument

........................................................................
& : Mitglied der gleichen Patentfamilie, übereinstimmendes
 Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 22 19 5828

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-02-2023

| Im Recherchenbericht angeführtes Patentdokument | Datum der Veröffentlichung | Mitglied(er) der Patentfamilie | | Datum der Veröffentlichung |
|---|---|---|---|---|
| US 2011259333 A1 | 27-10-2011 | US | 2011259333 A1 | 27-10-2011 |
| | | US | 2014034056 A1 | 06-02-2014 |
| WO 2020092701 A2 | 07-05-2020 | CN | 113382676 A | 10-09-2021 |
| | | EP | 3873329 A2 | 08-09-2021 |
| | | JP | 2022506805 A | 17-01-2022 |
| | | US | 2022020488 A1 | 20-01-2022 |
| | | WO | 2020092701 A2 | 07-05-2020 |

Für nähere Einzelheiten zu diesem Anhang : siehe Amtsblatt des Europäischen Patentamts, Nr.12/82

EPO FORM P0461